(19) Europäisches Patentamt
European Patent Office
Office européen des brevets

(11) Publication number: **0 011 067**
**B1**

(12) # EUROPEAN PATENT SPECIFICATION

(45) Date of publication of patent specification: 21.04.82

(21) Application number: 78300183.7

(22) Date of filing: 21.07.78

(51) Int. Cl.³: C 07 D 337/14, C 07 D 409/04, C 07 D 409/06, C 07 D 417/04, C 07 D 417/06, A 61 K 31/38

(54) **Prostaglandin antagonists, their production and their pharmaceutical compositions.**

(30) Priority: 26.07.77 US 819199
23.06.78 US 917212

(43) Date of publication of application:
28.05.80 Bulletin 80/11

(45) Publication of the grant of the patent:
21.04.82 Bulletin 82/16

(84) Designated Contracting States:
BE CH DE FR GB LU NL SE

(56) References cited:
FR - A - 2 312 241

(73) Proprietor: MERCK & CO. INC.
126, East Lincoln Avenue P.O. Box 2000
Rahway, New Jersey 07065 (US)

(72) Inventor: Rokach, Joshua
416 Canterbury Place
Chomedey-Laval (CA)
Inventor: Rooney, Clarence Stanley
2902 Hickory Hill Drive
Worcester Pennsylvania 19440 (US)
Inventor: Cragoe, Edward Jethro
2211 Oak Terrace Drive
Lansdale Pennsylvania 19446 (US)

(74) Representative: Crampton, Keith John Allen et al,
D YOUNG & CO 10 Staple Inn
London WC1V 7RD (GB)

EP 0 011 067 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Prostaglandin antagonists, their production and their pharmaceutical compositions

This invention relates to prostaglandin antagonists. Prostaglandin antagonists are useful in treating a variety of conditions, such as allergic asthma where excessive contractile activity of prostaglandins and prostaglandin biosynthetic intermediates occur.

The prostaglandin antagonists of the present invention are 7- and 8-substituted-dibenzo[b,f]thiepins having the structural formula:

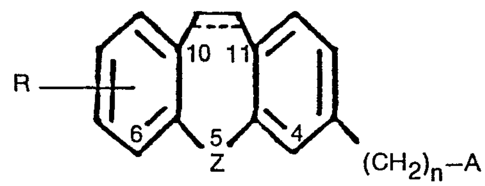

in which *n* is 0 or an integer from 1 to 4; Z is thio, sulfinyl, or sulfonyl; R is in the 7- or 8-position and is hydrogen, halogen including chlorine, bromine, fluorine and iodine, amino, $C_{1-4}$ alkyl, $C_{1-4}$ alkanoyl, hydroxyl, $C_{1-4}$ alkoxy, mercapto, $C_{1-4}$ alkylthio, $C_{1-4}$ alkylsulfinyl, $C_{1-4}$ alkylsulfonyl, trifluoromethyl, trifluoromethylthio, cyano, carboxy, nitro, $C_{1-4}$ alkylamino or di($C_{1-4}$ alkyl)amino; A is 5-tetrazolyl, 3-hydroxy-1,2,5-thiadiazol-4-yl, 4-hydroxy-2,5-dioxo-$\Delta^3$-pyrrolin-3-yl or

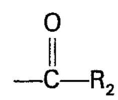

where $R_2$ is hydroxy, $C_{1-4}$ alkoxy, N,N-di($C_{1-4}$ alkyl)amino-($C_{1-4}$ alkoxy), $C_{1-4}$ hydroxyalkoxy, carboxy-($C_{1-4}$ alkoxy), amino, $C_{1-4}$ alkylamino, di($C_{1-4}$ alkyl)amino, $C_{1-4}$ alkylsulfonylamino, carboxy($C_{1-4}$ alkyl)amino, carbamoyl($C_{1-4}$ alkyl)amino or 2-imino-3-methylthiazolidine, and the dotted line indicates either an olefinic bond or saturation at the 10-, 11-position; and the pharmaceutically acceptable salts thereof.

As used herein, the term, halogen (or halo), means chlorine, bromine, iodine or fluorine. Unless otherwise specifically stated, the term lower alkyl and loweralkoxy means straight and branched chain alkyl and alkoxy groups having 1 to 4 carbon atoms in the alkyl or alkoxy groups having 1 to 4 carbon atoms in the alkyl or alkoxy residue, for example, methyl, ethyl, *n*-propyl, isopropyl, *n*-butyl, methoxy, ethoxy, *n*-propoxy and isobutyoxy. The term loweralkanoyl includes straight or branched chain alkanoyl groups having 1 to 4 carbon atoms in the alkanoyl residue, for example, formyl, acetyl, propanoyl, isobutyryl and isobutyryl.

These dibenzo[b,f]thiepin derivatives antagonize the actions of contractile prostaglandins, such as $PGF_2$, $PGG_2$ and $PGH_2$ and $TXA_2$. The use of agents that act as prostaglandin antagonists offers new approaches to therapy in a number of disease states. For example, certain prostaglandins, such as $PGF_{2\alpha}$, $PGG_2$ and $PGH_2$, are potent contractants of bronchial muscle. Indeed human asthmatics have been shown to be especially sensitive to the bronchial constricting action of $PGF_{2\alpha}$.

In addition to the involvement of contractile prostaglandins in chronic obstructive lung disease (or asthma), prostaglandins are known to play a role in other allergic conditions, as well as inflammation, diarrhoea, hypertension, angina, platelet aggregation, cerebral spasm, premature abortion, and dismenorrhoea.

In addition to the prostaglandin antagonist actions, the dibenzo[b,f]thiepins of this invention are antagonists of slow-reacting substance of anaphylaxis (SRS—A). This contractile substance is released in the lung tissue in allergic asthma, and antagonism of its actions contributes to alleviation of this disease.

The dibenzo[b,f]thiepins of this invention are prepared according to the following general reaction scheme.

An appropriately substituted mercaptobenzoic acid II is reacted with m-dibromobenzene III ($R_3$=Br) to obtain the O-(3-bromophenyl)benzoic acid IV. Or alternatively, an appropriately substituted o-bromobenzoic acid II ($R_2$=Br) is reacted with m-bromobenzenethiol III ($R_3$=SH) to give IV,

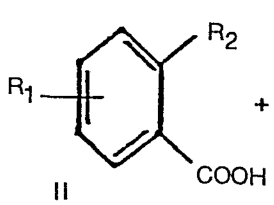

II

+

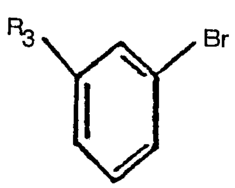

III

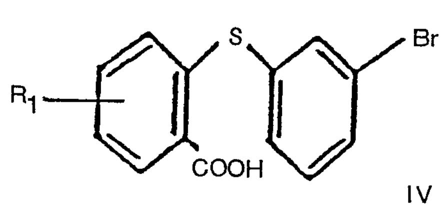

IV

where $R_1$ is hydrogen, nitro, amino, $C_{1-4}$ alkanoyl, hydroxyl, $C_{1-4}$ alkoxy, $C_{1-4}$ alkylsulfinyl, $C_{1-4}$ alkylsulfonyl, $C_{1-4}$ alkyl, trifluoromethyl or trifluoromethylthio. $R_2$ and $R_3$ are different: one of them is mercapto and the other is bromine.

Generally, the sulfide-forming reaction is carried out according to the methods described by Jilek et al., Monatsh. Chem. 96, 200 (1965), Protiva et al, Czech. Patent 121,377 C.A. 68 (105, 247t, 1968) and U.S.P. 3,711,489, and by other procedures well known in the art.

The resulting o-(3-bromophenylthio)benzoic acid (IV) is reduced to the alcohol, brominated, and the bromo replaced with cyano. The cyano derivative is then hydrolyzed to the carboxylic acid V.

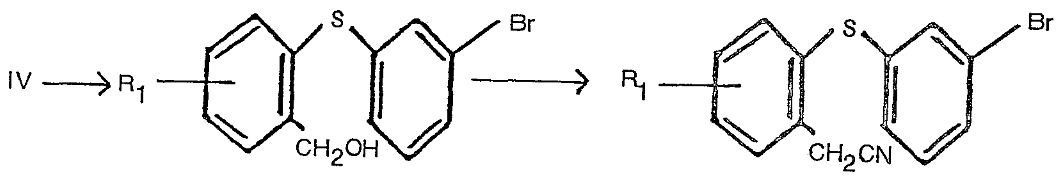

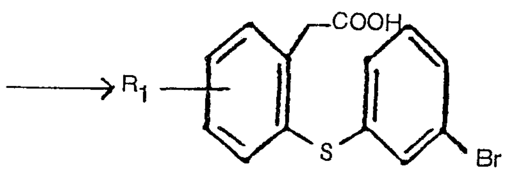

V

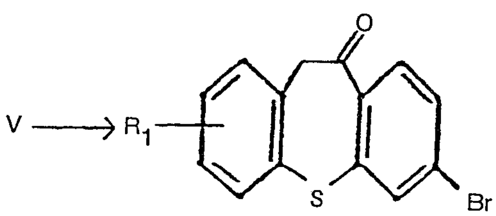

VI

The carboxylic acid V is transformed into the 3-bromo-11-oxo-10,11-dihydrodibenzo[b,f]thiepin by first conversion to the acid halide with thionyl or phosphoryl halide followed by Friedel-Crafts cyclization with a Lewis acid such as aluminium chloride to give VI. Reduction of the ketone VI with alkali metal borohydrides, followed by heating with catalytic amounts of a mineral acid, such as sulfuric acid or toluenesulfonic acid, provides the 3-bromodibenzo[b,f]thiepin VII.

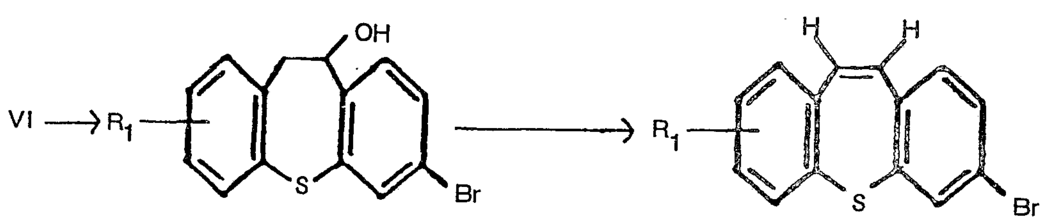

VII

The 3-bromo derivative VII is then converted to the 3-nitrile VIII by reaction with cuprous cyanide in a high boiling polar solvent such as dimethylformamide or N-methylpyrrolidone.

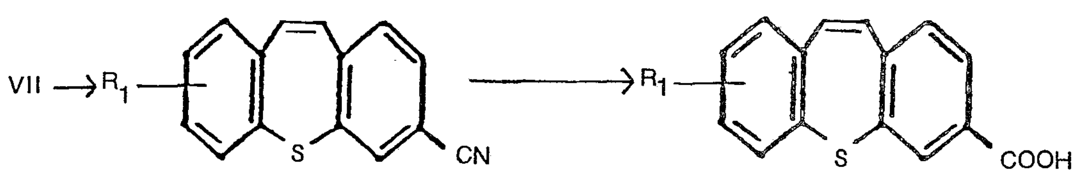

VIII IX

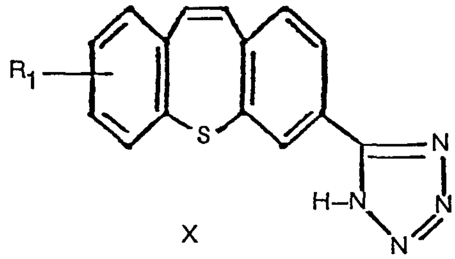

X

The 3-cyano derivative VIII may be hydrolyzed with aqueous mineral acid or base to give the dibenzo[b,f]thiepin-3-carboxylic acid IX. The 3-cyano compound VIII may also be reacted with azide ion at reflux in an inert solvent such as dimethylformamide or hexamethylphosphorictriamide for 1/4 to 18 hours to give the tetrazole derivative X. Alternatively, the cyano intermediate VIII may be oxidized with organic peroxides such as peroxy acids, for example, m-chloroperbenzoic acid, in a stepwise fashion to the corresponding sulfoxide XI and sulfone XII, controlling the molar ratio of oxidant to reductant. This determines the oxidation level of the sulfur. For example, a 1:1 molar ratio results largely in the production of sulfoxide XI. In contrast, a 2 to 3 molar excess of oxidant results in a yield predominantly comprising the sulfone XII.

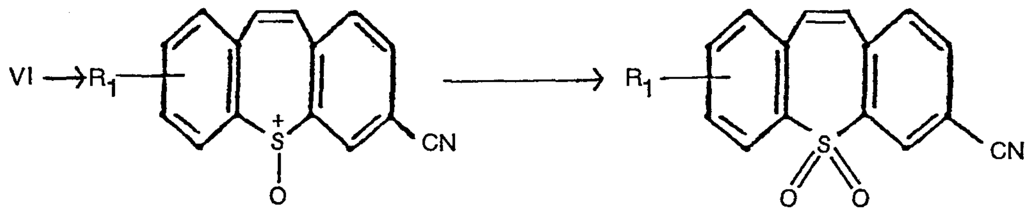

XI XII

Hydrolysis of XI and XII using aqueous mineral acid or alkali provides the corresponding carboxylic acids XIII and XIV.

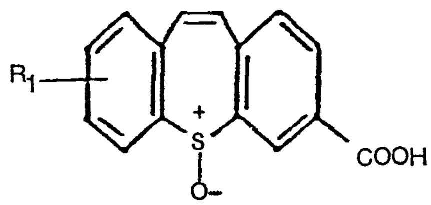

XIII

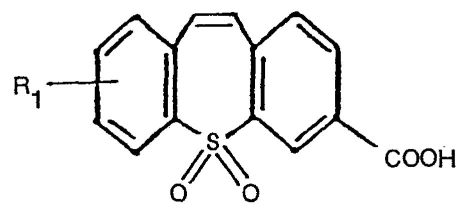

XIV

Reaction of XI and XII with azide ion as described above provides the tetrazoles XV and XVI, respectively.

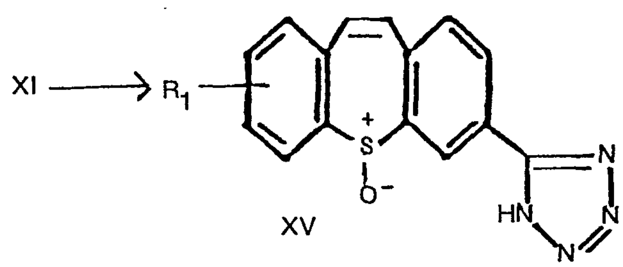

XV

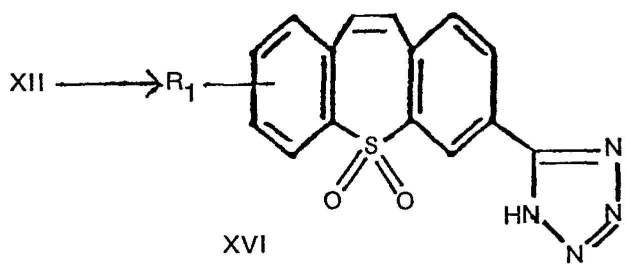

XVI

Compounds of type I where the 10—11 double bond is saturated are prepared from intermediate VI in which the 3-bromo is converted to a cyano derivative XVII, then hydrolyzed with mineral acid to the acid XVIII and esterified to the ester XIX, where R′ is loweralkyl.

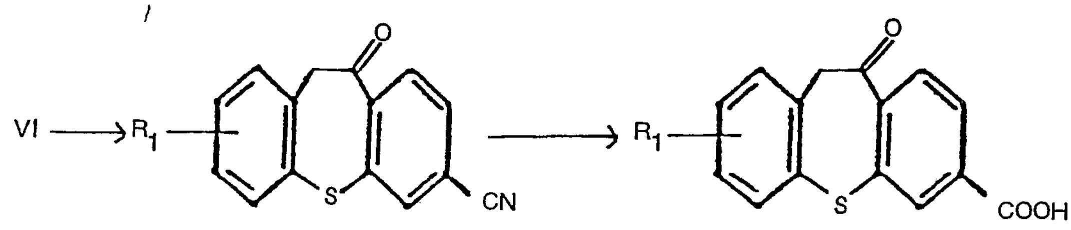

XVII XVIII

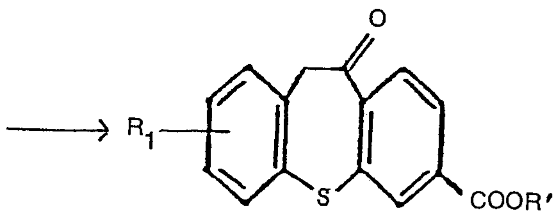

XIX

The ester XIX is reduced by conventional methods, e.g., Woff-Kishner, to compound XXII or better by reduction with $NaBH_4$ to XX followed by $PBr_3$ reaction to XXI, then reduction with $NaBH_4$ in solvent such as sulfolane to XXII.

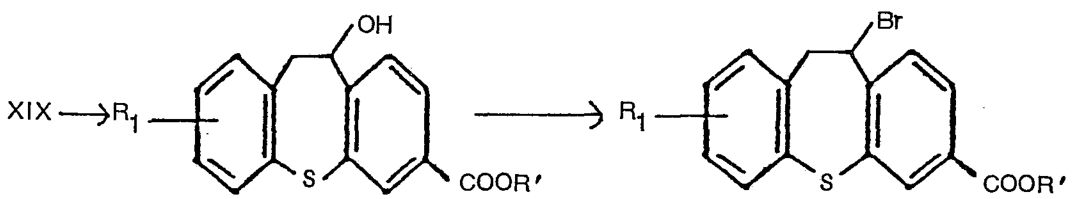

XX XXI

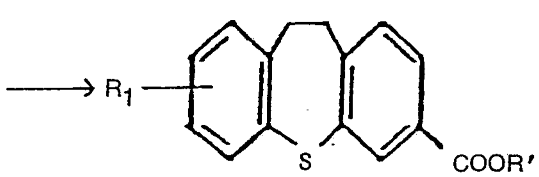

XXII

Compound XXII may be hydrolyzed with aqueous mineral acid or base to give the 10,11-dihydro[b,f]thiepin-3-carboxylic acid XXIII. Compound XXII may also be oxidized with one equivalent of organic peroxides, such as peroxy acids, for example, m-chloroperbenzoic acid and the like, to yield the sulfoxide XXIV, which can then be hydrolyzed with mineral acid or base to provide the carboxylic acid XXV.

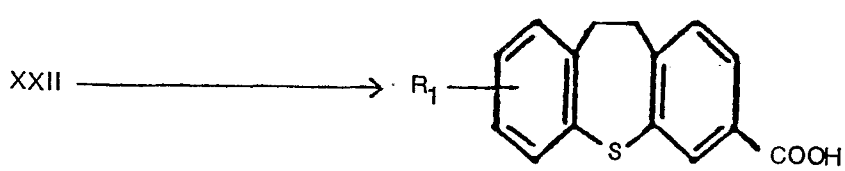

XXIII

XXII 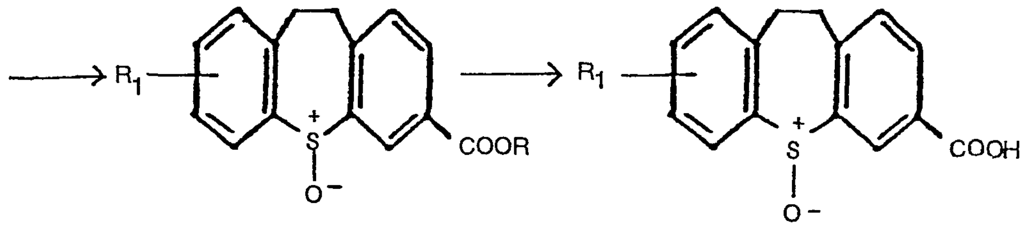

XXIV XXV

The acid XXIII may be oxidized with excess organic peroxides such as hydrogen peroxide in acidic solvents such as acetic acid at temperatures ranging from 0°—100°C. to yield compound XXVI.

XXIII 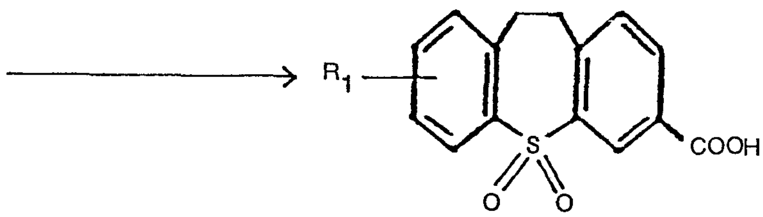

XXVI

Compound VI may be reacted with phosphorus tribromide followed by reduction with $NaBH_4$ in solvents, such as sulfolane, and reaction with cuprous cyanide in high boiling polar solvents, such as dimethylformamide, N-methylpyrrolidone, to yield XXVII.

VI 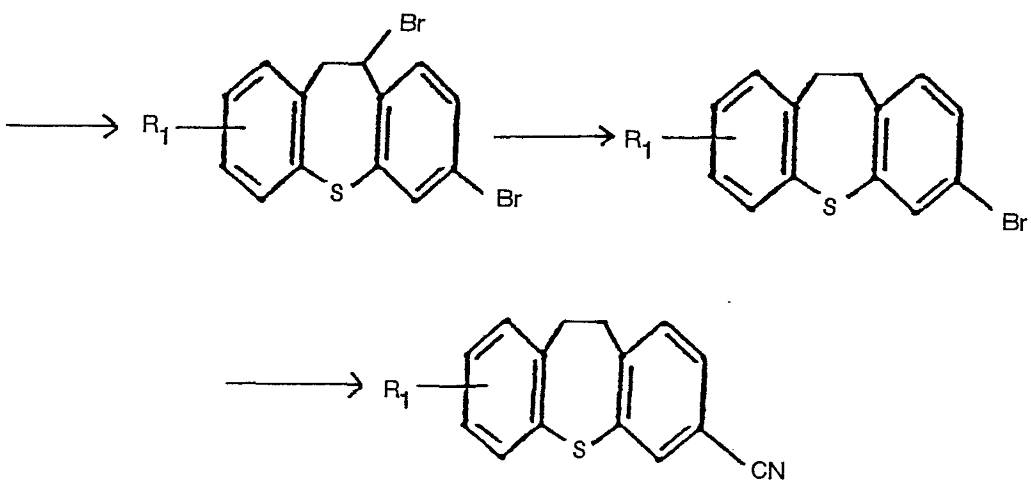

XXVII

The 3-cyano derivative XXVII may be reacted with azide ion at reflux in an inert solvent such as dimethylformamide or in THF with the addition of Lewis acid, such as $AlCl_3$, to yield the tetrazole derivative XXVIII. Compound XXVII can also be oxidized with excess peroxy acid, such as m-chloroperbenzoic acid followed by the tetrazole forming reaction to yield XXIX.

XXVII 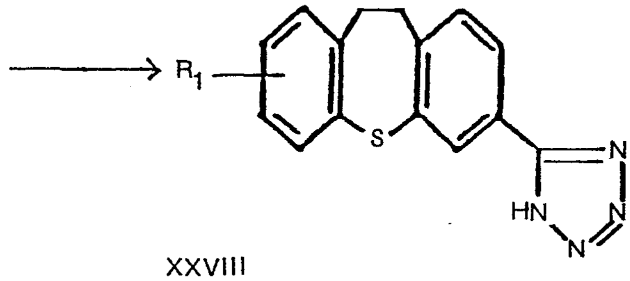

XXVIII

XXVII

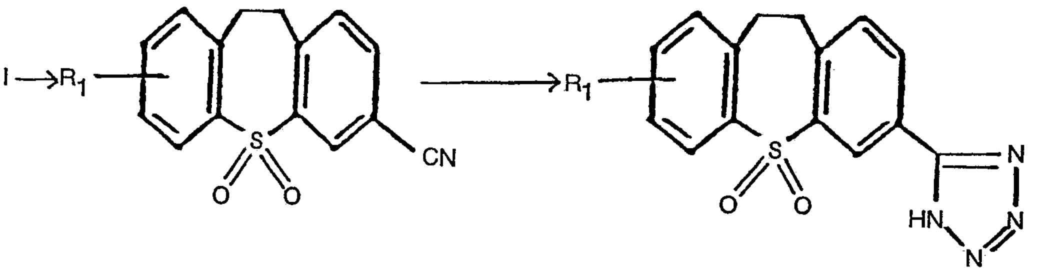

XXIX

Tetrazole XXVIII may be oxidized with peroxides such as hydrogen peroxide in acidic medium, such as acetic acid, to yield compound XXX.

XXVIII

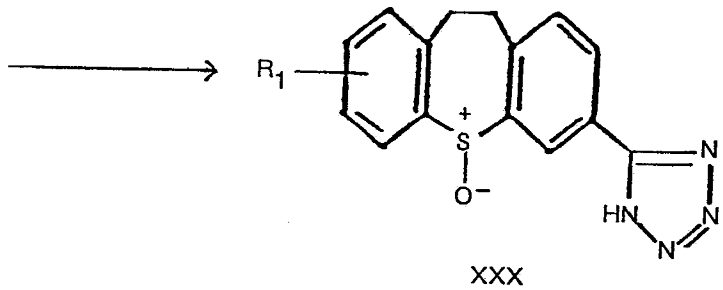

XXX

XVII may also be oxidized with one equivalent of organic peroxide, such as peroxy acids, for example, m-chloroperbenzoic acid followed by mineral acid or base hydrolysis to yield XXXI.

XVII

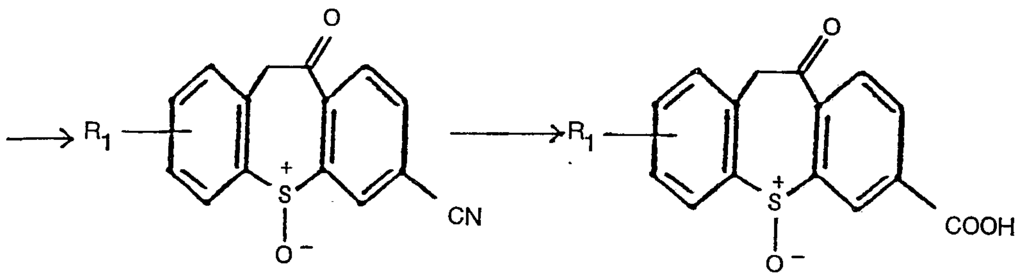

XXXI

XVII can also be reacted with azide ion at reflux in an inert solvent such as dimethylformamide or in THF with the addition of a Lewis acid such as $AlCl_3$ to yield the tetrazole XXXII.

XVII

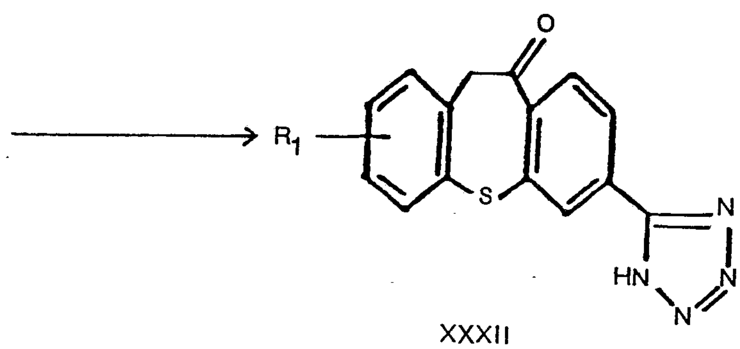

XXXII

Substituent R in I can also be introduced by modification of the nitro group in VIII ($R=NO_2$) and XXVII ($R=NO_2$) by known procedures. For example, XXXIII can be reduced with stannous chloride in acidic medium, e.g. hydrochloric acid, to yield XXXIV which can be hydrolysed with mineral acids or bases to XXXV.

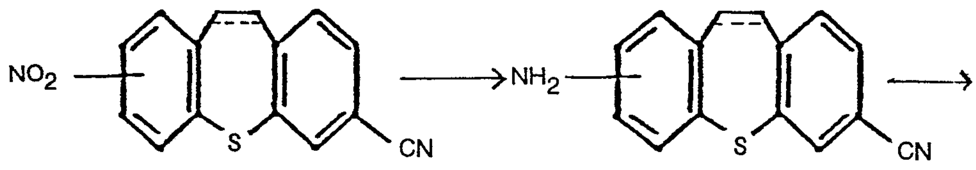

XXXIII XXXIV

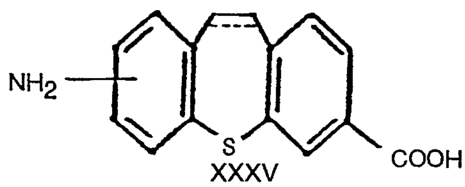

XXXV

Alternatively, XXXIII may be oxidized with peroxides, for example, m-chloroperbenzoic acid to yield XXXVI which can be reduced to XXXVII and then hydrolysed with mineral acids or bases to XXXVIII.

XXXIII

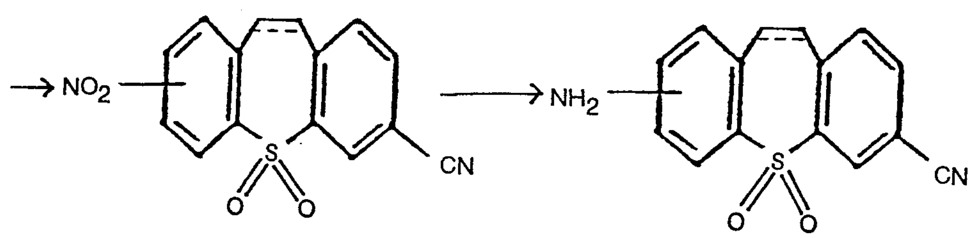

XXXVI XXXVII

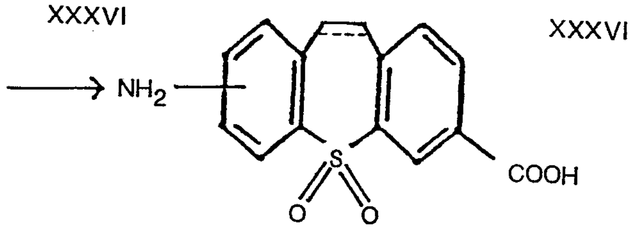

XXXVIII

Intermediate XXXIV can be reacted with sodium nitrite in mineral acid to the diazonium salt XXXIX, where X is a mineral acid counter ion, for example, $Cl^-$, $HSO_4^-$ or $BF_4^-$, which on reaction with CuCl and $CuCl_2$ yields intermediate XL which can be hydrolysed to the acid XLI. Intermediate XL may also be oxidized to the sulfone derivative, then followed by a hydrolysis to the carboxylic acid XLII.

XXXIV

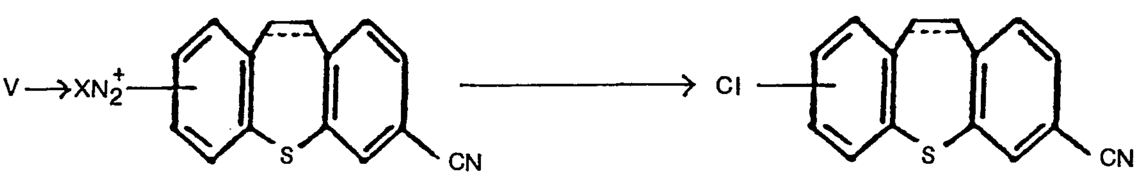

XXXIX XL

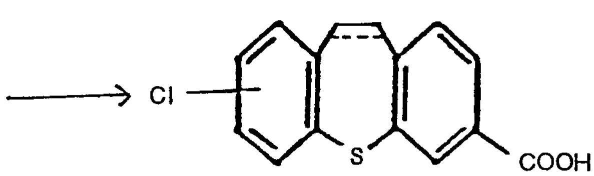

XLI

XL

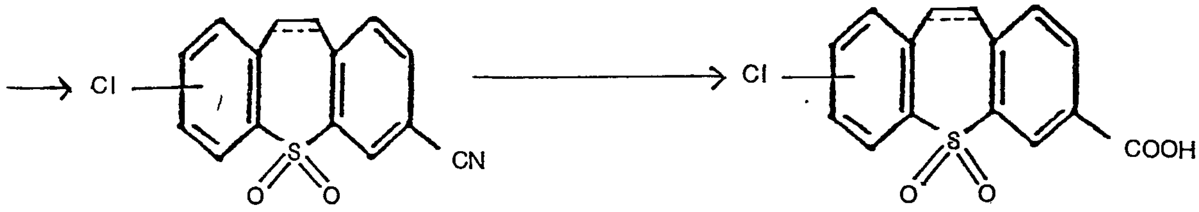

XLII

Derivative XXXIX can be hydrolyzed with a solution of sulfuric acid 10 to 50% in strength at temperatures ranging from 0°—90°C. to yield XLIII. XXXIX may also be reacted with potassium thioxanthate at temperatures from 40°—70°C. followed by basic hydrolysis to yield the thiol acid XLIV.

XXXIX

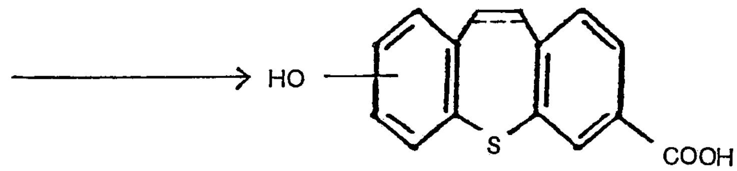

XLIII

XXXIX

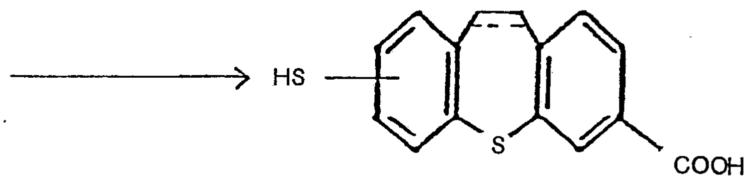

XLIV

Compound XXXVII can be transformed in the usual manner to the diazonium slat XLV. XLV can be reacted as described above to yield compounds XLVI and XLVII.

XXXVII

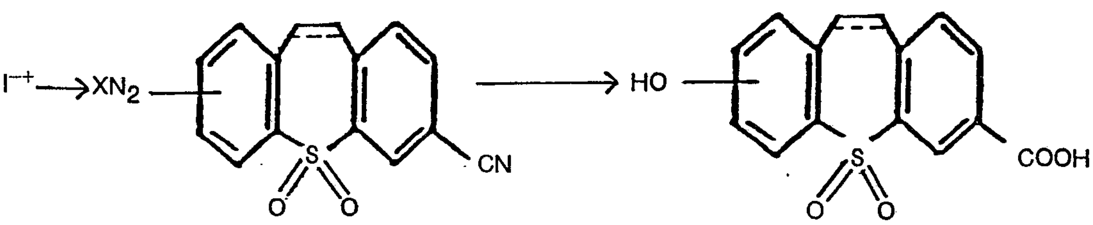

XLV

XLVI

XLV

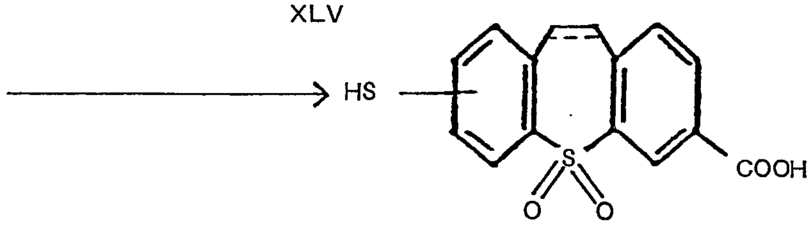

XLVII

Compounds XLIII, XLIV, XLVI, XLVII can be reacted with alkyl halides RX in which R is a lower alkyl $C_1$ to $C_4$, benzyl, and X is a leaving group such as Cl, Br, I,

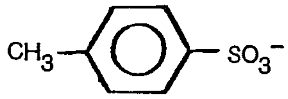

in the presence of bases such as alkali metal carbonates or hydroxides, in solvents such as dimethylformamide, at temperatures ranging from 30°—160°C. to yield XLVIII, XLIX, L and LI, respectively.

XLIII 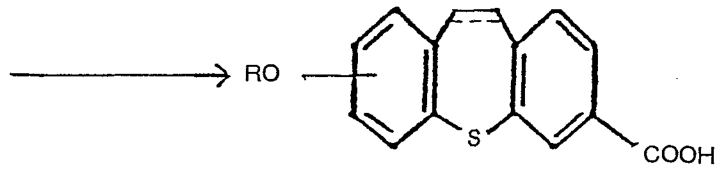

XLVIII

XLIV 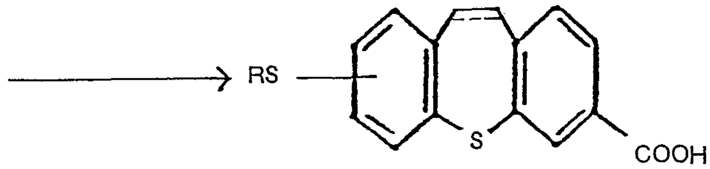

XLIX

XLVI 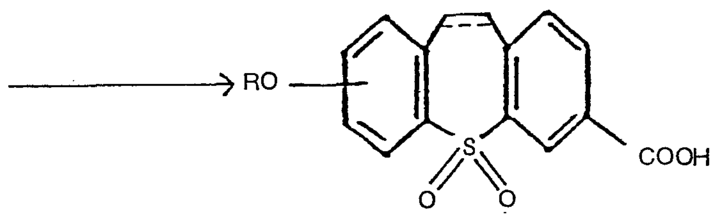

L

XLVII 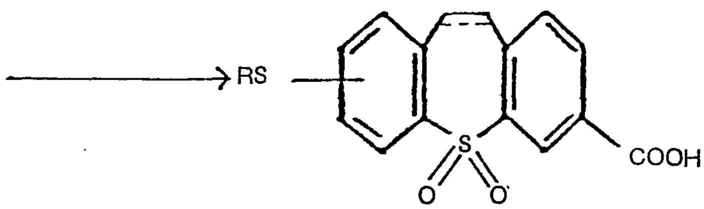

LI

Compound XLIX can in a controlled oxidation with peroxides such as hydrogen peroxide or organic peroxy acids such as m-chloroperbenzoic acid, yield compound LII. LI may be oxidized with one equivalent of organic peroxides such as m-chloroperbenzoic acid or with hydrogen peroxide in hydroxylic solvents such as alcohols, organic acids such as acetic acid, at temperatures below 30°C., to yield LIII. Compounds XLIX, LI and LIII may also be oxidized with excess organic peroxides such as m-chloroperbenzoic acid at room temperature, or with peroxides such as hydrogen peroxide in acidic medium such as acetic acid at temperatures between 80° and 100°C. to yield LIV.

XLIX 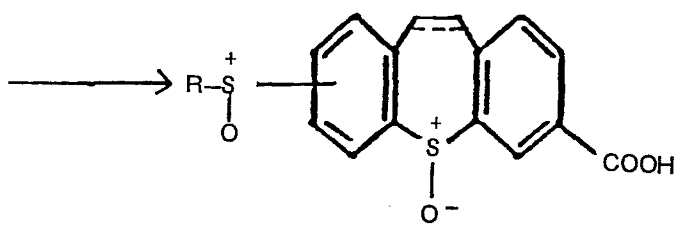

LII

LI 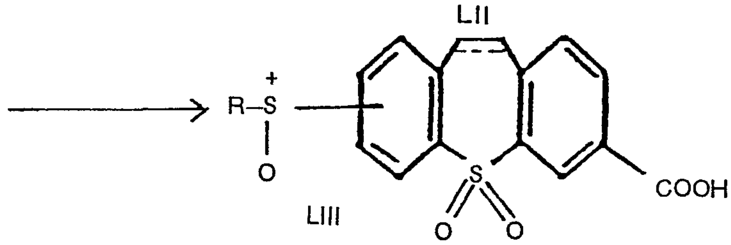

LIII

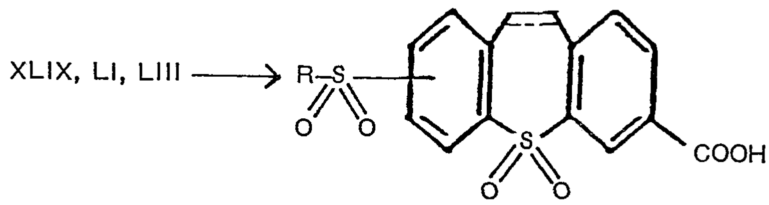


LIV

Specific introduction of substituents in position 8 in I can also be achieved. For example, XXVII ($R_1$=H) can react with alkanoyl halide RCOX or alkanoic anhydride RCOOCOR in which R is a lower alkyl $C_1$ to $C_4$ and X is chloro or bromo, to yield the substituted acyl LV which upon acid or base hydrolysis affords acid LVI.

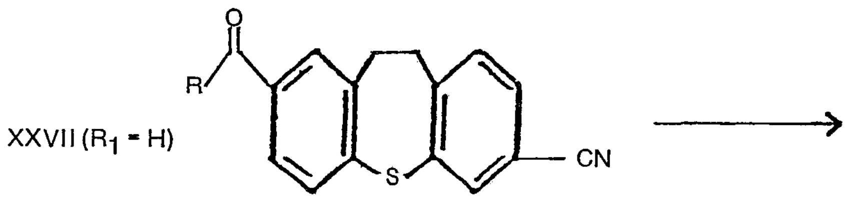


LV

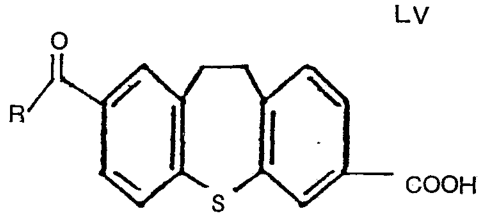

LVI

LV can be oxidized with oxidizing agents such as inchloro perbenzoic acid stepwise to yield sulfoxide LVII and sulfone LVIII which are hydrolyzed under acidic or basic conditions to afford acids LIX and LX respectively.

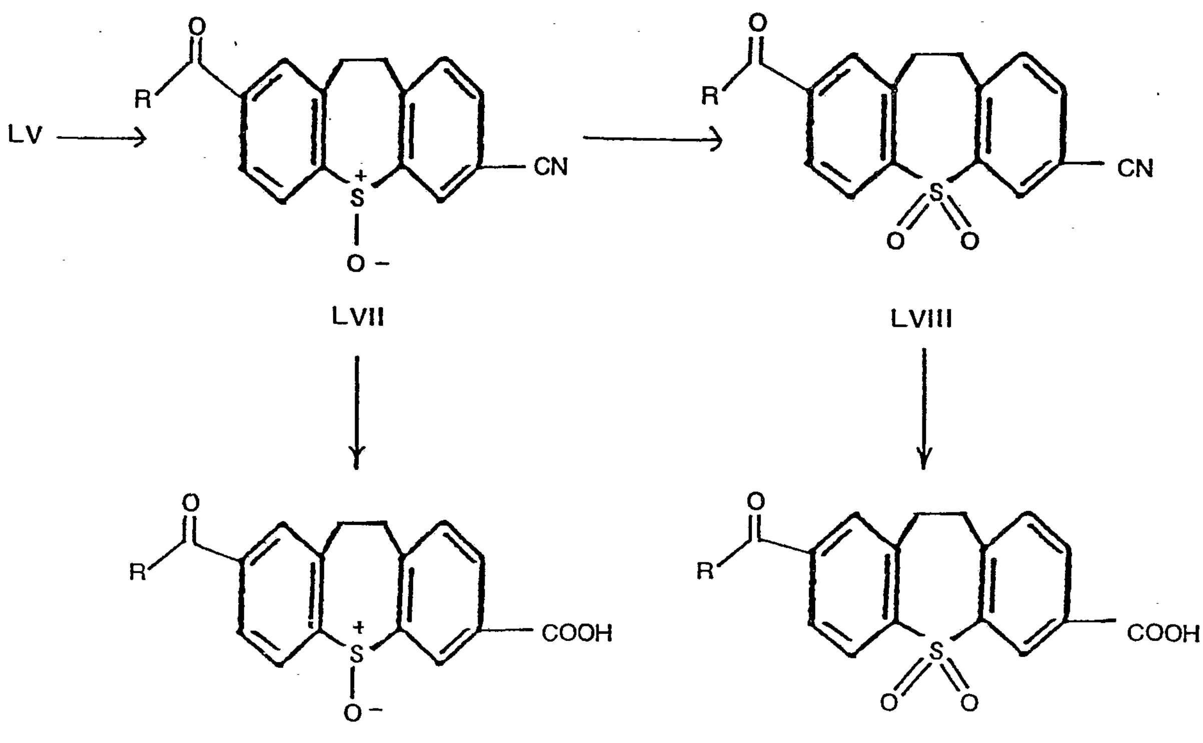


LVII

LVIII

LIX

LX

Compound LVI can be reacted with hydroxylamine hydro chloride with presence of base to yield oxime LXI which on a Beckman rearrangement, yields the acylamino compound LXII which upon hydrolysis yields amino acid LXIII.

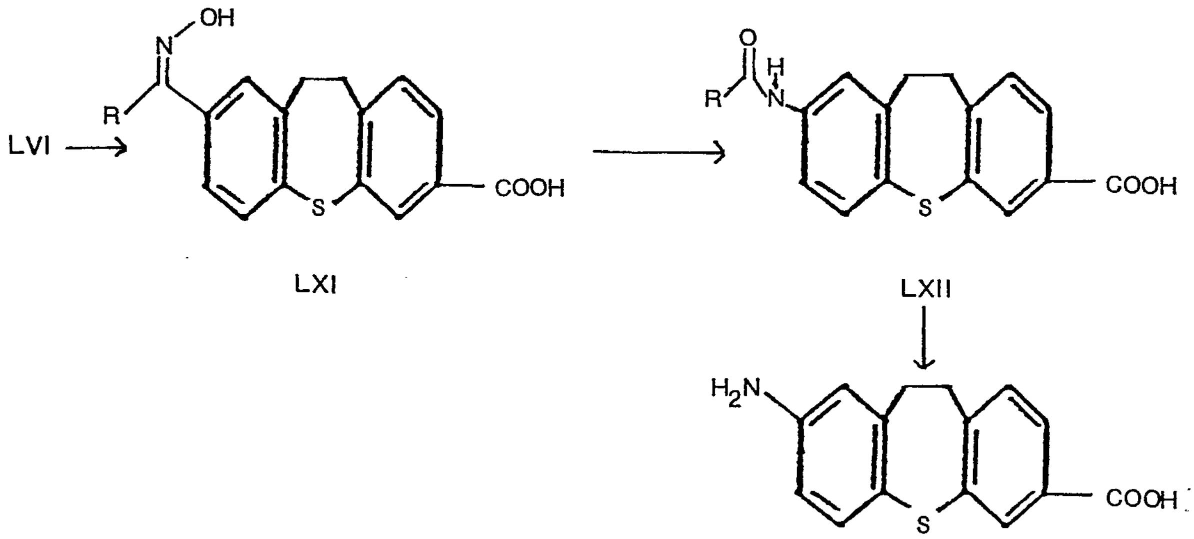

LVI

LXI

LXII

LXIII

Compounds LXII can be oxidized with hydrogen peroxide in acetic acid stepwise to yield the corresponding sulfoxide LXIV and sulfone LXV which upon hydrolysis afford the acids LXVI and LXVII.

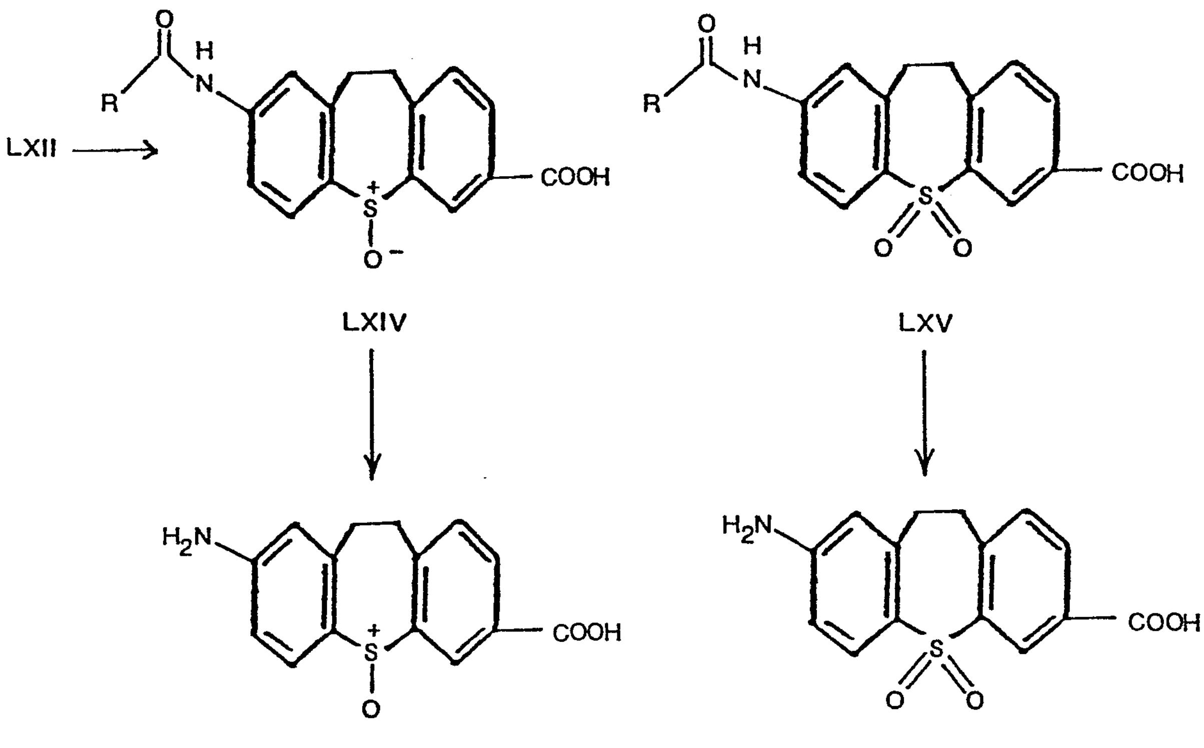

LXII

LXIV

LXV

LXVI

LXVII

Compounds LXIII, LXVI, LXVII can be treated in various Sandmeyer reactions as described earlier to yield I substituted in the 8 position.

Compound LVI ($R=CH_3$) when treated with sodium hypochlorite and base at temperatures from 0°—70° for half an hour yield the diacid LXVIII. When the process is carried for 2 days under the same conditions LXIX is obtained.

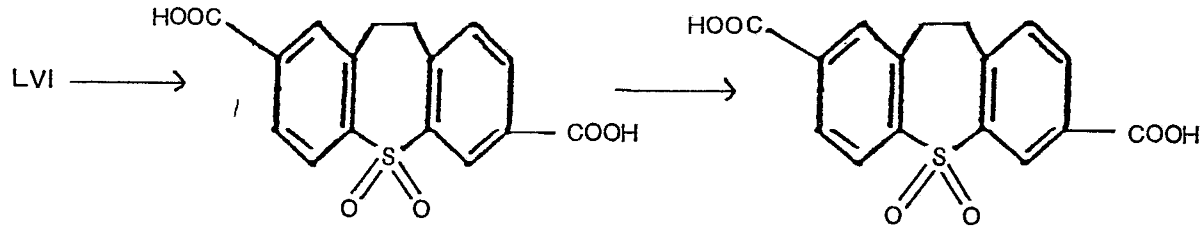


LXVIII LXIX

Compounds LVI, LIX, LX can be reduced with sodium borohydrid to afford the corresponding alcohols, LXX, LXXI, LXXII.

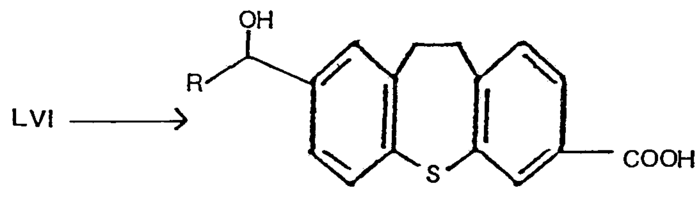


LXX

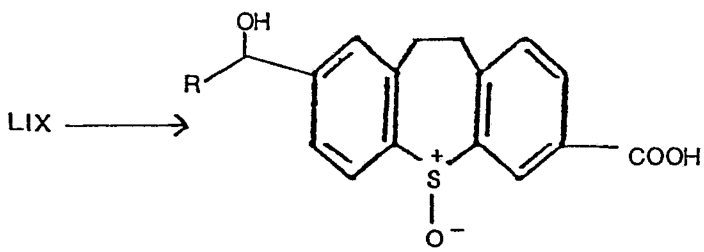


LXXI

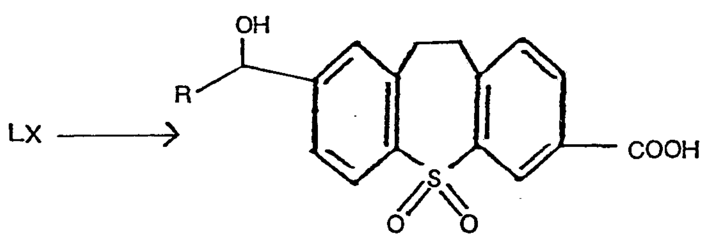


LXXII

It will be obvious to those skilled in the art that the nitrile, XVII, can be substituted for the nitrile starting material, XXVII, in the foregoing reaction sequences in order to prepare correspondingly substituted 10,11-dihydro-11-oxodibenzo[b,f]thiepins.

In addition to their therapeutic properties as noted above, the 3-carboxylic acid derivatives of this invention serve as valuable intermediates in the preparation of other variously substituted thiepins of formula I. Thus, for example, the 3-carboxylic acid of formula XVIII ($R_1$=R as defined in formula I) may be converted readily into the corresponding acid halide, preferably the acid chloride, by treating the carboxylic acid with a thionyl halide, preferably thionyl chloride. The resulting 3-halocarbonyl 10,11-dihydro-11-oxodibenz[b,f][1,4]thiepin i.e., the 3-chlorocarbonyl compound of formula LXXIII then may be treated with various well-known reagents to form desired ester and amide derivatives. These reactions are illustrated in the following reaction scheme wherein R is as previously defined, it being understood that they are equally applicable to the 3-carboxylic acids of formula IX or XXIII.

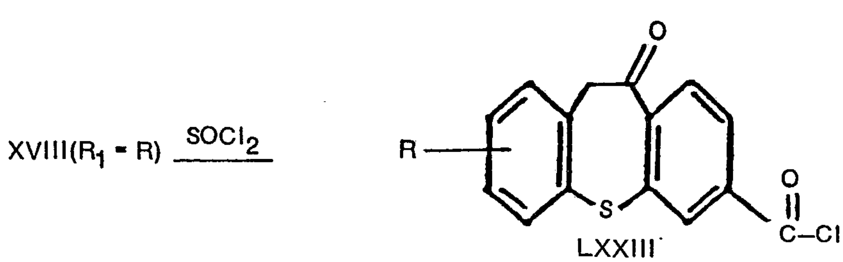


LXXIII

Thus, for example, the chlorocarbonyl compound of formula LXXIII may be treated:

(a) with a loweralkanol such as, for example, methanol, ethanol, 2-propanol, butanol and 2-butanol, to form the corresponding loweralkyl ester, LXXIV:

LXXIII

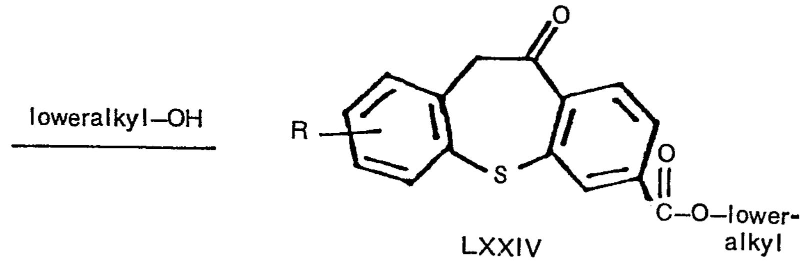


LXXIV

(b) with ammonia to form the corresponding carboxamide, LXXV:

LXXIII

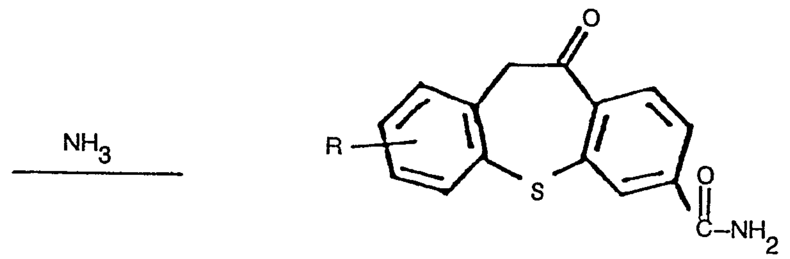


LXXV

(c) with an N-loweralkylamine such as for example, methylamine, ethylamine, propylamine, isopropylamine and butylamine, or an N,N-diloweralkylamine such as, for example, dimethylamine, diethylamine, dipropylamine and dibutylamine, to form the corresponding N-loweralkylcarboxamide LXXVI or N,N-diloweralkylcarboxamide, LXXVII:

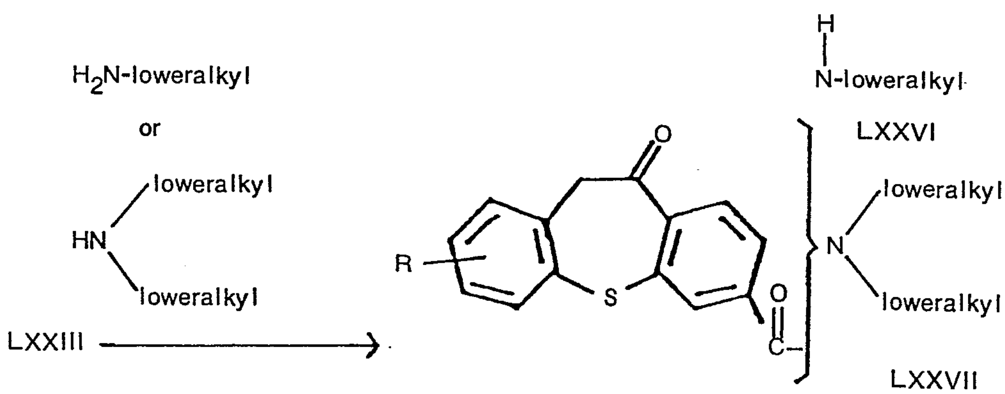


LXXIII

LXXVI

LXXVII

(d) with a loweralkylsulphonamide such as, for example, methane sulphonamide, ethane sulphonamide, propane sulphonamide and butane sulphonamide, to form the corresponding N-loweralkylsulfonylcarboxamide, LXXVIII:

LXXIII

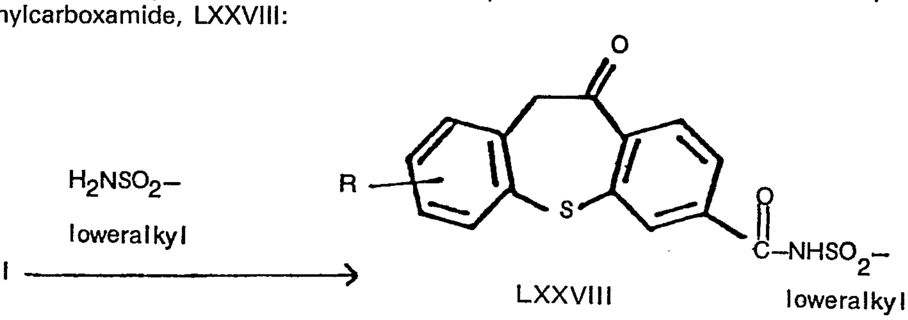


LXXVIII

(e) with 2-imino-3-methylthiazolidine to form the corresponding (3-methyl-2-thiazolidinylidene)-carboxamide, LXXIX:

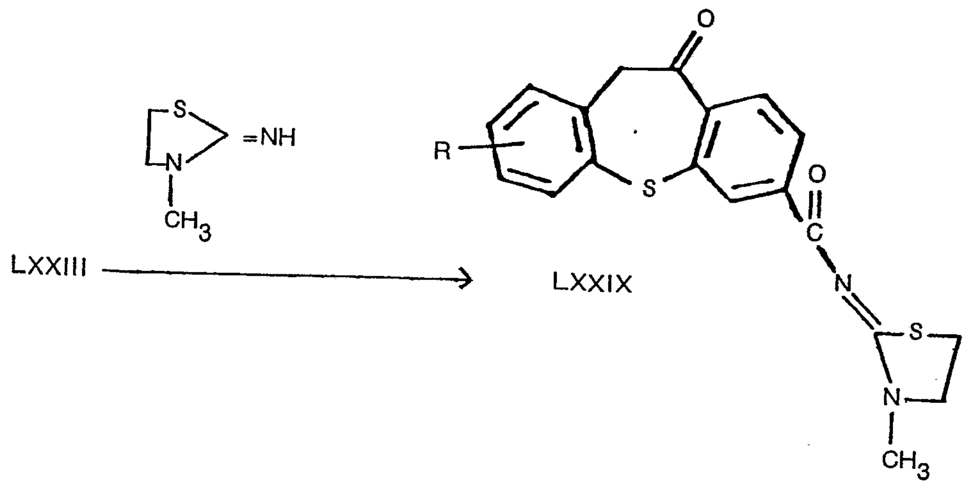

LXXIII → LXXIX

(f) with a loweralkyldiol such as, for example ethylene glycol, trimethylene glycol and 1,4-butanediol, to form the corresponding hydroxyloweralkylester, LXXX:

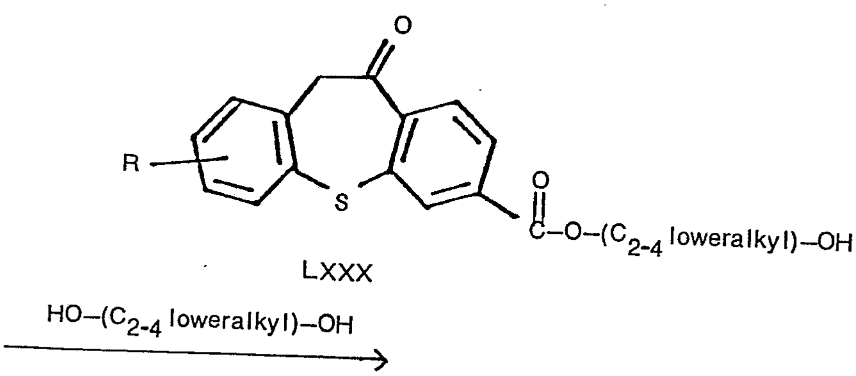


LXXX

LXXIII →

(g) with an N,N-diloweralkylaminoloweralkanol such as, for example, N,N-dimethylethanolamine, N,N-diethylethanolamine, 3-N,N-dimethylaminopropan-1-ol and 4-N,N-diethylaminobutan-1-ol, to form the corresponding N,N-diloweralkylaminoloweralkyl ester, LXXXI:

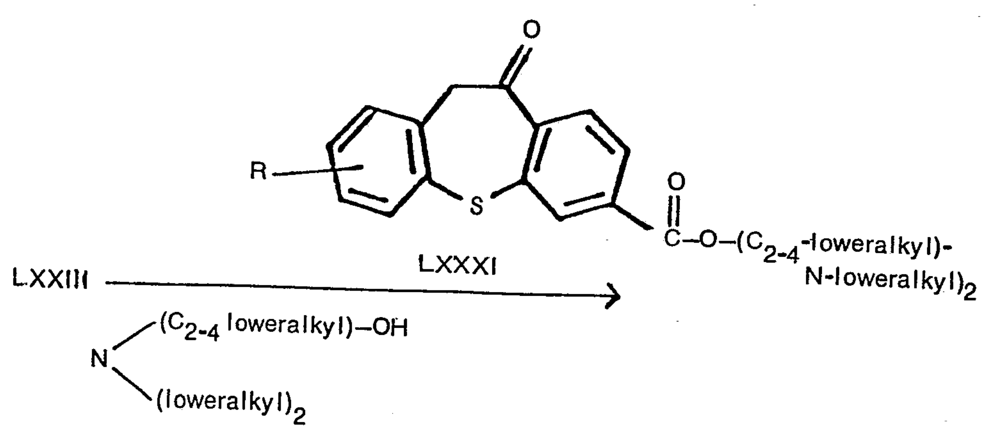

LXXIII → LXXXI

(h) with an amino acid such as, for example, glycine, alanine and valine, to form the corresponding N-carboxyloweralkylcarboxamide, LXXXII:

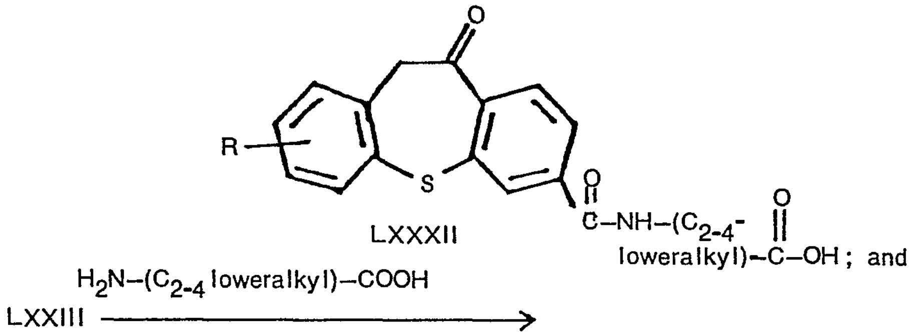


LXXIII → LXXXII ; and

(i) with an alkali metal salt of a hydroxyloweralkanoic acid such as, for example, hydroxyacetic acid, 3-hydroxybutyric acid and β-hydroxypropionic acid, to form the corresponding carboxyloweralkyl ester, LXXXIII:

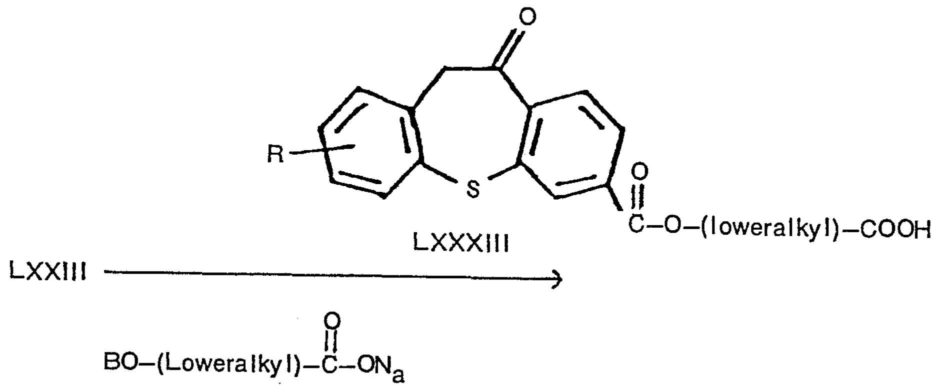


LXXIII → LXXXIII

Where the corresponding sulfinyl or sulfonyl derivatives are desired, the corresponding 11-oxide or 11,11-dioxide 3-carboxylic acid may be substituted for starting material XVIII in the foregoing reaction sequence. Alternatively, it will be clear to those skilled in the art that the product esters and amides obtained in the foregoing reaction sequence may be oxidized by the techniques already described to obtain the corresponding sulfinyl or sulfonyl derivatives.

Those thiepins of this invention wherein the substituent at the 3-position is 3-hydroxy-1,2,5-thiadiazol-4-yl are prepared by refluxing a 3-cyano intermediate (a compound of formula XVII where $R_1$=R as defined in formula I, for example) in formic acid in the presence of Raney nickel alloy for 1 to 2 hours in order to obtain the corresponding 10,11-dihydro-11-oxodibenzo-[b,f]-thiepin-3-carboxaldehyde. The aldehyde product then is converted into the corresponding 3-(2-aminoacetonitrile) by treatment with sodium cyanide in an alcoholic solvent saturated with ammonia and in the presence of ammonium chloride and ammonium hydroxide. The reaction usually is conducted at room temperature and requries from 8 to 16 hours for completion. The aminoacetonitrile so produced is treated with concentrated hydrochloric acid at room temperature for 20 to 45 minutes in order to obtain the corresponding 3-(2-aminoacetamide) which then is treated with sulfur monochloride in dimethylformamide to obtain the desired 10,11-dihydro-11-oxo-dibenzo[b,f]thiepin-3-(3-hydroxy-1,2,5-thiadiazol-4-yl) of formula LXXXIV. This reaction sequence is illustrated in the following diagram.

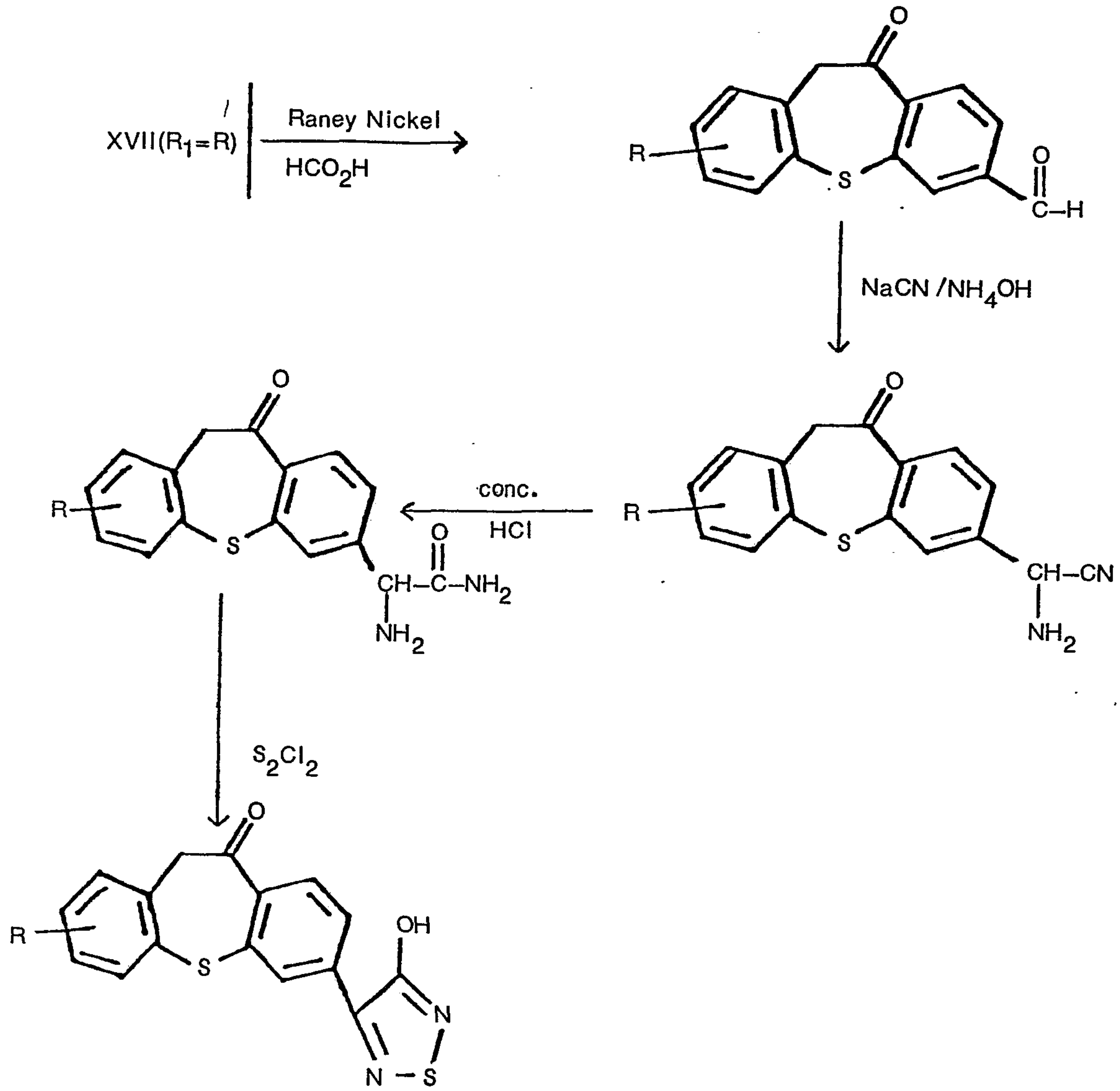


LXXXIV

The novel thiepins of this invention wherein the substituent at the 3-position is 4-hydroxy-$\Delta^3$-pyrroline-3-yl-2,5-dione are prepared from the appropriately substituted 3-carboxylic acid by reducing the acid to the corresponding alcohol with borane in tetrahydrofuran. The reaction conveniently is carried out at room temperature under an inert atmosphere and usually requires 2 to 4 hours for completion. The alcohol then is brominated with phosphorous tribromide and the bromomethyl compound so produced is treated with sodium cyanide to form the corresponding 3-cyanomethyl derivative. These reactions may be carried out at room temperature and usually require from 1 to 3 hours for completion. The cyanomethyl intermediate then is hydrolyzed to the corresponding acetic acid which is treated with thionyl chloride followed by ammonia to form the corresponding 3-acetamide derivative by techniques already described. The acetamide then is treated with diethyloxalate in dimethylformamide in the presence of potassium *t*-butoxide to form the desired dibenzo[b,f]thiepin-3-(4-hydroxy-$\Delta^3$-pyrrolin-3-yl-2,5-dione), LXXXV. This reaction sequence is illustrated in the diagram below.

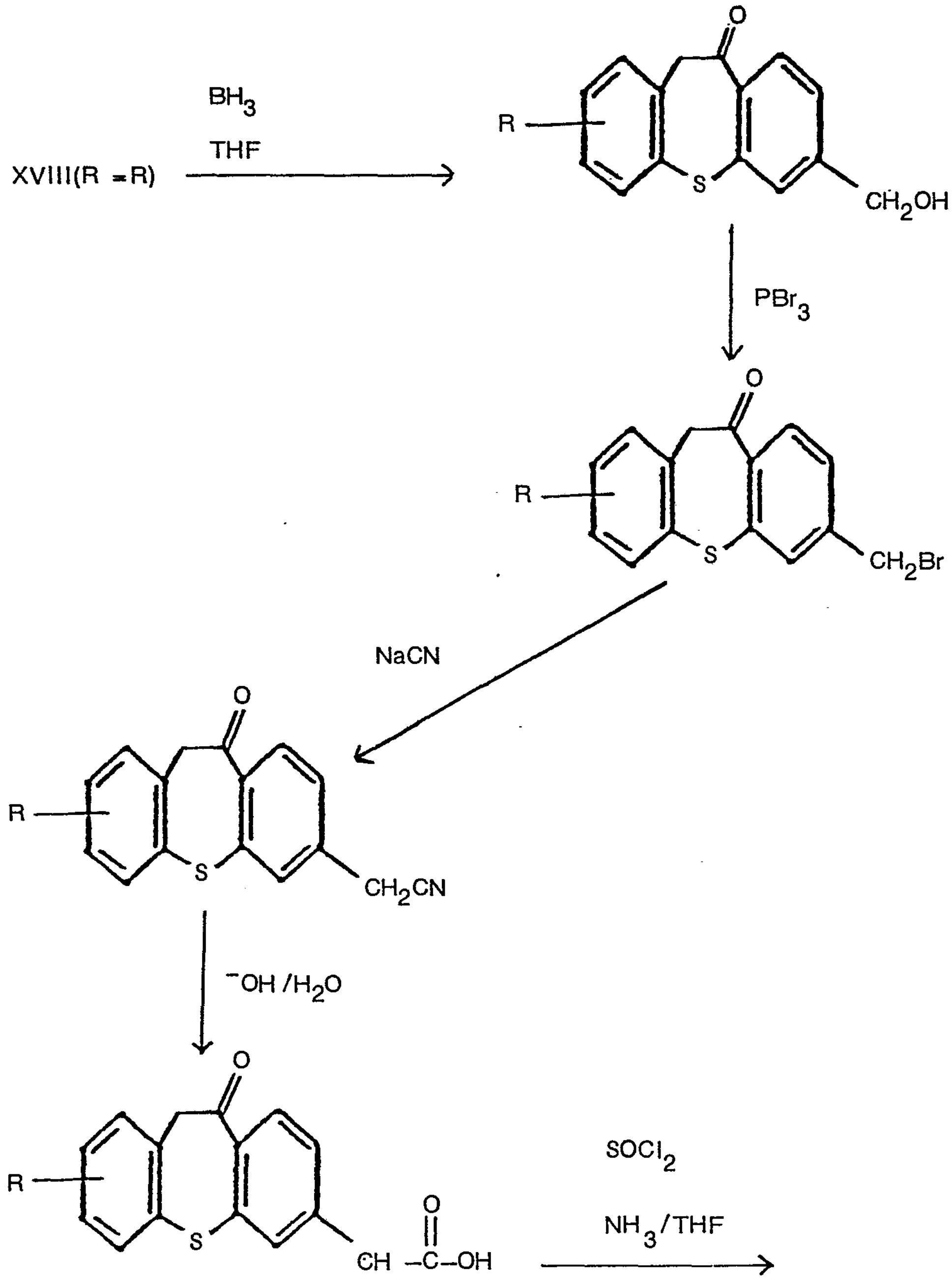
XVIII(R =R)
BH3
THF
PBr3
NaCN
⁻OH /H2O
SOCl2
NH3/THF

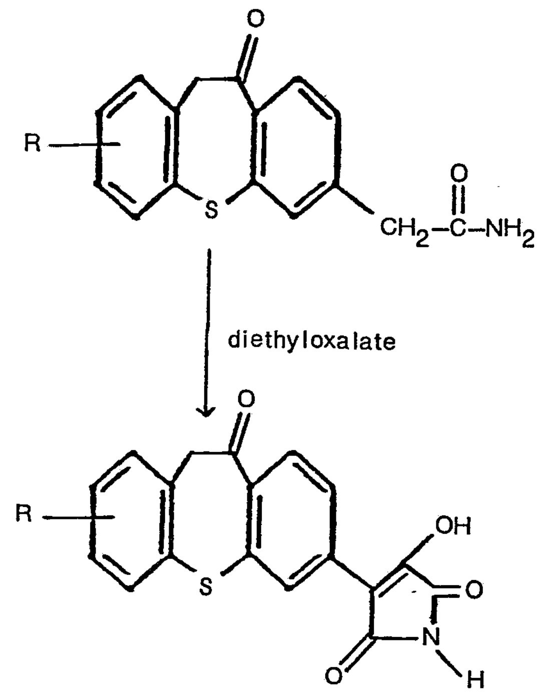


LXXXV

Where corresponding sulfinyl or sulfonyl derivatives are desired, the products of the four reaction schemes described immediately above may be oxidized by the techniques already described.

It will be noted that the reaction sequence described above affords not only thiepins of this invention wherein the substituent at the 3-position is 4-hydroxy-$\Delta^3$-pyrroline-3-yl-2,5-dione, but leads also to the preparation of those thiepins of this invention wherein the substituent at the 3-position is a loweralkanoic acid (i.e., compounds of formula I wherein A is

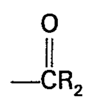

n is an integer between 1 and 4 and $R_2$ is hydroxy). Thus, starting with the appropriately substituted 3-carboxylic acid, reduction, bromination, cyanization and oxidation in that order afford the corresponding 3-acetic acid derivative. Quite obviously, the described reduction, bromination, cyanization and oxidation sequence can be repeated, employing the 3-acetic acid derivative as starting material, in order to obtain the corresponding propionic acid derivative which, in turn, can be employed as starting material for preparing the corresponding butyric acid derivative. In this manner, any desired 3-loweralkanoic acid derivative of the instant invention readily is prepared. Corresponding sulfinyl or sulfonyl derivatives are prepared by the oxidation techniques previously described.

The 3-cyanoloweralkyl intermediates obtained from the reduction, bromination and cyanization steps described above also serve as intermediates in the preparation of other therapeutically active thiepins of formula I. Thus, for example, an appropriately substituted 3-cyanomethyl-10-11-oxodibenzo[b,f]thiepin may be treated with sodium azide and ammonia by techniques previously described to form the corresponding 3-(1H-tetrazol-5-ylmethyl)-10,11-dihydro-11-oxo-dibenzo[b,f]thiepin and the product, if desired, can be oxidized to form the corresponding sulfinyl or sulfonyl derivative.

As noted above, pharmaceutically acceptable salts of the novel thiepins also are included within the scope of this invention. The term, pharmaceutically acceptable salts, is intended to include salts derived from pharmaceutically acceptable non-toxic acids and bases such as, for example, ammonium salts, alkali metal salts such as sodium and potassium salts, alkaline earth metal salts such as magnesium and calcium salts, salts of organic bases such as amine salts derived from mono-, di and tri-loweralkyl or loweralkanoyl amines such as trimethylamine, dimethylamine and triethanolamine, salts derived from heterocyclic amines such as piperidine, pyridine, piperazine and morpholine, and salts derived from pharmaceutically acceptable acids such as hydrochloric acid, sulfuric acid, tartaric acid and propionic acid.

Example 1

STEP 1

2-(3-Bromophenylthio)benzoic Acid

A mixture consisting of 179 g. m-dibromobenzene (0.758 mole); 46.6 g. thiosalicyclic acid (0.303 mole); 25.9 g cuprous oxide (0.181 mole); 212 cc. quinoline; and 24 cc. pyridine is mechanically stirred and heated in an oil bath at 200°C to 210°C. for three hours. The internal temperature remains constant at 145°C. The reaction mixture is then poured into 1500 cc. of 5N aqueous HCl. The oily solid is filtered, then dissolved in 750 cc. 1N aqueous NaOH solution; this solution is filtered through celite, then extracted three times with ether. The aqueous fraction is acidified with concentrated HCl and the resulting grayish solid filtered, washed well with water and dried. The crude yield obtained is 65 g. (69.4%). It is used as such in the next step.

STEP 2

2-(3-Bromophenylthio)benzyl Alcohol

A solution of 63.3 g. of 2-(3-bromophenylthio)benzoic acid (0.205 mole, crude) in 400 cc. dry tetrahydrofuran (THF) is treated dropwise at room temperature and under nitrogen atmosphere with 240 cc. 0.96N borane in THF. Hydrogen evolution is noticed during approximately one-third of the addition. After completion of the addition, the mixture is left stirring for one additional hour, then decomposed by the dropwise addition of 15 cc. water. Most of the THF is evaporated off and the residue is partitioned ether and water. The organic phase contains the crude alcohol which is chromatographed on a column of silica gel (1 kg.); elution is done with a mixture of 20% ethyl acetate in benzene. Pure 2-(3-bromophenylthio)-3-nitrobenzyl alcohol (50.03 g.) is obtained as a yellow oil (82.8% yield).

STEP 3

2-(3-Bromophenylthio)benzyl Bromide

To 50.03 g. 2-(3-bromophenylthio)benzyl alcohol (0.17 mole) cooled in an ice bath is added dropwise 6 cc. phosphorus tribromide (0.06 mole). The resulting milky oil is stirred in the cold an additional 15 minutes then decomposed with ice water. Extraction with ether affords 58.6 g. of 2-(3-bromophenylthio)benzyl bromide as a yellowish-brown oil which is used as such in the next step.

STEP 4

2-(3-Bromophenylthio)benzyl Cyanide

12.4 g. sodium cyanide (0.25 mole) is added to a solution of 58.6 g. 2-(3-bromophenylthio)benzyl bromide (0.164 mole) in 200 cc. dimethylformamide (DMF). The reaction is slightly exothermic. The resulting solution is stirred for an hour without cooling, diluted with a large volume of water and extracted with ether three times. Ethereal extracts are washed several times with water and dried over sodium sulfate and the ether is evaporated off. The crude yield of 2-(3-bromophenylthio)benzyl cyanide is 49.6 g.; the product is an oil which is hydrolysed as such.

STEP 5

2-(3-Bromophenylthio)phenyl Acetic Acid

49.6 g. crude 2-(3-bromophenylthio)benzyl cyanide is refluxed in a mixture of 650 cc. denatured alcohol for three hours. The solution is concentrated and the sodium salt of the acid separates. The mixture is diluted to three liters with water and acidified with concentrated HCl. The free acid precipitates and is filtered. The yield of crude 2-(3-bromophenylthio)phenyl acetic acid is 49.3 g. (93.5%). It is used as such in the next step.

STEP 6

3-Bromo-10,11-dihydro-11-oxodibenzo[b,f]thiepin

49.3 g. (0.1526 mole) crude 2-(3-bromophenylthio)phenyl acetic acid and 50 cc. thionyl chloride are refluxed together for 10 minutes. The excess of thionyl chloride is evaporated off, the residual oil dissolved in 1,2-dichloroethane and the mixture evaporated again to remove the last traces of thionyl chloride.

The oily acid chloride is dissolved in 400 cc. 1,2-dichloroethane and 22.4 g. aluminum chloride (10% excess) is added in portions. The reaction is slightly exothermic but no cooling is necessary. The reaction is permitted to go for 40 minutes then the mixture is poured onto ice. The organic fraction is collected and the aqueous fraction extracted three times with chloroform. Combined organics are washed with water, dried over sodium sulfate and stripped to dryness. The solid residue is triturated in ether and filtered, then triturated in methanol and filtered. The yield of crude product is 34.8 g. (74.8%).

STEP 7

3-Bromo-10,11-dihydro-11-hydroxydibenzo[b,f]thiepin

17 g. of 3-bromo-10,11-dihydro-11-oxodibenzo[b,f]thiepin (0.056 mole) is dissolved in a mixture of 150 cc. DMF and 150 cc absolute alcohol. 1.7 g. sodium borohydride (0.045 mole) is added and the

mixture is left stirring overnight at room temperature. The ethanol is evaporated and the residual DMF solution is diluted with water and extracted with ether three times. The ethereal extracts are washed several times with water, dried and evaporated to a thick oil. A quantitative yield of the alcohol is obtained.

STEP 8

3-Bromodibenzo[b,f]thiepin

17 g. 3-bromo-11-hydroxy-10,11-dihydrodibenzo[b,f]thiepin is dissolved in 600 cc. benzene; 1 gram *p*-toluene sulfonic acid is added and the mixture is refluxed with elimination of water for two hours. The solution is cooled and washed with aqueous sodium bicarbonate solution and water, dried and stripped to a solid residue. 16 g. of 3-bromodibenzo[b,f]thiepin is obtained.

STEP 9

3-Cyanodibenzo[b,f]thiepin

A mixture containing 15.4 g. 3-bromodibenzo[b,f]thiepin and 7.16 g. cuprous cyanide (50% excess) in 100 cc. DMF is refluxed for 10 hours. The dark mixture is cooled to 10°C. and poured onto 500 cc. 20% aqueous HCl solution. Solids are filtered and washed well with water, then dissolved in chloroform. The solution is filtered through celite to remove insoluble cuprous salts, then evaporated to a solid residue. Chromatography on silica gel, eluting with benzene, affords 7.3 g. pure 3-cyanodibenzo[b,f]thiepin as a yellow solid, m.p. 108°C.—110°C. Yield is 58.3%.

STEP 10

Dibenzo[b,f]thiepin-3-carboxylic Acid

2.0 g. 3-cyanodibenzo[b,f]thiepin are refluxed in a mixture of 25 cc. concentrated HCl and 25 cc. glacial acetic acid for 19 hours; the acid separates from the hot solution. After cooling, the mixture is diluted with water and the light yellow solid is filtered and washed well with water. The yield of dibenzo[b,f]thiepin-3-carboxylic acid is 2.07 g. (95.8%), m.p. 251°C.—254°C.

| | | | | | | | |
|---|---|---|---|---|---|---|---|
| Calculated | : | C: | 70.85; | H: | 3.95; | S: | 12.61 |
| Found | : | C: | 71.05; | H: | 3.88; | S: | 12.35. |

Example 2

STEP 1

3-Cyanodibenzo[b,f]thiepin-5-oxide

5 g. 3-cyanodibenzo[b,f]thiepin (0.021 mole) is dissolved in 300 cc. methylene chloride and 3.45 g. *m*-chloroperbenzoic acid (0.020 mole) added in portions. The solution is stirred for two hours at room temperature, then excess of calcium hydroxide is added. The mixture is stirred for a few minutes and filtered through celite, the filtrate is evaporated down and the residue chromatographed on silica gel, eluting with a 50:50 mixture of chloroform and benzene. A small amount of starting material is recovered, and the yield of sulfoxide is 4.92 g. (92%), light yellow solid, m.p. 220°C.—222°C.

STEP 2

Dibenzo[b,f]thiepin-3-carboxylic Acid 5-Oxide

925 mg 3-cyanodibenzo[b,f]thiepin 5-oxide is refluxed for 3 1/2 hours in a mixture of 60 cc. 10% aqueous sodium hydroxide solution and 60 cc. denatured alcohol. The mixture is diluted with 1/2 liter of water and acidified with concentrated HCl. The acid precipitates and is filtered and dried. The yield of white dibenzo[b,f]thiepin-3-carboxylic acid 5-oxide is 965 mg. (97%), m.p. dec. 249°C.

| | | | | | | | |
|---|---|---|---|---|---|---|---|
| Calculated | : | C: | 66.75; | H: | 3.73; | S: | 11.86. |
| Found | : | C: | 66.75; | H: | 3.62; | S: | 11.67. |

Example 3

STEP 1

3-Cyanodibenzo[b,f]thiepin-5,5-dioxide

5.4 g. 3-cyanodibenzo[b,f]thiepin (0.023 mole) is dissolved in 300 cc. methylene chloride and 15.87 g. *m*-chlorperbenzoic acid (0.092 mole) are added in portions. The resulting solution is stirred at room temperature for one hour, then excess of calcium hydroxide is added. The mixture is filtered through celite and the filtrate is stripped to dryness. The yellow solid is triturated in benzene and filtered. 5.46 g. (89%) of the sulfone, m.p. 229°C.—231°C. is obtained.

STEP 2

Dibenzo[b,f]thiepin-3-carboxylic Acid 5,5-Dioxide

13.6 g. 3-cyanodibenzo[b,f]thiepin-5,5-dioxide is refluxed for 2.5 hours in a mixture of 20 cc. 10% aqueous sodium hydroxide solution and 20 cc. ethanol. The mixture is diluted with water and acidified with concentrated HCl. The acid precipitates as a white solid. The yield of acid, m.p. 268°C.—270°C. is 1.38 g. (94.6%).

| | | | | | | |
|---|---|---|---|---|---|---|
| Calculated | : | C: | 62.93; | H: | 3.52; | S: 11.20. |
| Found | : | C: | 62.72; | H: | 3.58; | S: 10.91. |

Example 4

3-(5-Tetrazolyl)dibenzo[b,f]thiepin 5,5-Dioxide

A mixture made up of:

1 g. 3-cyanodibenzo[b,f]thiepin 5,5-dioxide (3.75 millimoles);

302 mg. sodium azide (4.65 millimoles);

273 mg. ammonium chloride (5.10 millimoles);

20 cc. DMF is refluxed for 16 hours. After cooling, the mixture is diluted with 10% aqueous sodium carbonate solution; the resulting solution is extracted three times with ether, thus affording 302 mg. recovered starting material. The aqueous fraction is acidified with concentrated HCl and the crude tetrazole (700 mg.) is filtered. It is chromatographed on a column of silica gel, eluting with a solvent mixture consisting of 4 parts methanol, 10 parts chloroform and 1 part 28% aqueous ammonium hydroxide by volume. The product is obtained from the column as the ammonium salt; it is dissolved in water and the solution is washed with chloroform. The aqueous fraction is then acidified with aqueous HCl solution and the tetrazole precipitates and is filtered and dried. The yield is 350 mg. (30%) of yellowish solid, m.p., dec. 247°C.

| | | | | | | | | |
|---|---|---|---|---|---|---|---|---|
| Calculated | : | C: | 58.06; | H: | 3.25; | N: | 18.05; | S: 10.32. |
| Found | : | C: | 58.29; | H: | 3.08; | N: | 18.17; | S: 10.11. |

Example 5

3-(5-Tetrazolyl)dibenzo[b,f]thiepin

A mixture consisting of 4 g. 3-cyanodibenzo[b,f]thiepin (17 millimoles); 1.8 g. sodium azide (28 millimoles); 1.63 g. ammonium chloride (30.5 millimoles) and 50 cc. DMF is refluxed for 17 hours. The mixture is cooled and diluted with aqueous sodium carbonate solution. The solution is extracted with ether, then acidified with aqueous HCl solution. The tetrazole precipitates and is filtered. Crystallization from methanol affords 3.93 g. (83%) yellow product, m.p. 213°C.—214°C.

| | | | | | | | | |
|---|---|---|---|---|---|---|---|---|
| Calculated | : | C: | 64.75; | H: | 3.59; | N: | 20.14; | S: 11.51. |
| Found | : | C: | 65.03; | H: | 3.71; | N: | 19.94; | S: 11.74. |

Example 6

3-(5-Tetrazolyl)dibenzo[b,f]thiepin-5-oxide

To a solution of 2.03 g. (7.3 millimoles) of 3-(5-tetrazolyl)dibenzo[b,f]thiepin in 100 cc. glacial acetic acid is added 15 cc. 30% aqueous hydrogen peroxide. The mixture is heated to 65°C. for 10 minutes; the sulfoxide separates from the hot solution. After cooling and diluting with water, the crystalline product is filtered and washed with water. It is then heated on a steam bath in 25 cc. DMF and filtered hot. The solid is washed with methanol and dried. The yield of sulfoxide is 1.55 g. (72%) as a white solid decompositing at 277°C.—279°C.

| | | | | | | | | |
|---|---|---|---|---|---|---|---|---|
| Calculated | : | C: | 61.21; | H: | 3.40; | N: | 19.03; | S: 10.09. |
| Found | : | C: | 60.93; | H: | 3.41; | N: | 19.08; | S: 10.66. |

Example 7

STEP 1

3-Cyano-10,11-dihydro-11-oxodibenzo[b,f]thiepin

36.45 g. 3-bromo-10,11-dihydro-11-oxodibenzo[b,f]thiepin and 32.11 g. cuprous cyanide are refluxed in 450 cc. dimethylformamide for five and one half hours. The reaction mixture is cooled down and poured into 2 l. of ice water with good mechanical stirring. The resulting solid is filtered, washed with water, then extracted into 1.7 l. chloroform. The insoluble copper salts are filtered and the filtrate evaporated to dryness. The residue is triturated in methanol and the insoluble yellow product is filtered and weighs 22.4 g. (74.6%), m.p. 166°—170°C.

STEP 2

10,11-Dihydro-11-oxodibenzo[b,f]thiepin-3-carboxylic Acid

20 g. of 3-cyano-10,11-dihydro-11-oxodibenzo[b,f]thiepin is refluxed in a mixture of 200 cc. concentrated hydrochloric acid and 200 cc. acetic acid for 21 hours. The desired acid precipitates out of the solution and is filtered while hot and washed with water and dried to yield 17.3 g. light yellow solid. From the mother liquors 2.74 g. of additional material is recovered, total yield 93.6%.

STEP 3

10,11-Dihydro-11-hydroxydibenzo[b,f]thiepin-3-carboxylic Acid

16.3 g. 10,11-dihydro-11-oxodibenzo[b,f]thiepin-3-carboxylic acid is stirred in aqueous sodium

bicarbonate solution and 5 g. sodium borohydride is added in portions. The foaming mixture is stirred for an additional half hour. The solution is extracted with ether, the aqueous layer acidified with hydrochloric acid and the solid filtered and dried to yield 15.8 g. of the desired yellow solid (96.2% yield), m.p. 202°—204°C.

| | | | | | | | |
|---|---|---|---|---|---|---|---|
| Calculated : | C: | 66.16; | H: | 4.44; | S: | 11.77. |
| Found : | C: | 66.00; | H: | 4.62; | S: | 11.54. |

STEP 4

Methyl-10,11-dihydro-11-hydroxydibenzo[b,f]thiepin-3-carboxylate

13.5 g. 10,11-dihydro-11-hydroxydibenzo[b,f]thiepin-3-carboxylic acid is suspended in ether and excess of diazomethane is added in portions. A clear solution results which is filtered from small particles in suspension and the filtrate evaporated to dryness to a yellow oil weighing 14.2 g.

STEP 5

Methyl 11-bromo-10,11-dihydrodibenzo[b,f]thiepin-3-carboxylate

14.7 g. methyl-10,11-dihydro-11-dihydroxydibenzo[b,f]thiepin-3-carboxylate is dissolved in 400 cc. ether, and 5 cc. phosphorus tribromide is added. The mixture is stirred at room temperature for 15 minutes. Ice is added and the mixture is filtered to yield a first crop of material. The organic layer in the filtrate is separated, dried and evaporated; the residue triturated with ether and filtered affords a second crop. The two crops are combined and dried to yield 15.47 g., m.p. 116°—123°C.

STEP 6

Methyl-10,11-dihydrodibenzo[b,f]thiepin-3-carboxylate

13.6 g. methyl-11-bromo-10,11-dihydrodibenzo[b,f]thiepin-3-carboxylate is suspended in 150 cc. sulfolane and to the mechanically stirred mixture 7.18 g. sodium borohydride is added in portions over one hour. The foamy mixture is stirred an additional half hour. The mixture is diluted with 1.5 l. of water and extracted with ether. The organic layer is washed with water, dried, evaporated, and the residual oil is chromatographed on silica gel. Elution with benzene affords 8.1 g. of the pure oily material.

STEP 7

10,11-Dihydrodibenzo[b,f]thiepin-3-carboxylic Acid

5.6 g. methyl-10,11-dihydrodibenzo[b,f]thiepin-3-carboxylate is refluxed in a mixture of 50 cc. 20% aqueous sodium hydroxide and 50 cc. ethanol until a clear solution results (15 to 20 minutes). Most of the ethanol is evaporated away and aqueous residue is diluted with water and acidified with hydrochloric acid. The white solid is collected, recrystallized from acetic acid to yield 4.125 g. (77.7%) of the desired white crystalline material, m.p. 196°—198°C.

| | | | | | | |
|---|---|---|---|---|---|---|
| Calculated : | C: | 70.29; | H: | 4.72; | S: | 12.51. |
| Found : | C: | 69.92; | H: | 4.71; | S: | 12.15. |

Example 8

STEP 1

Methyl 10,11-dihydrodibenzo[b,f]thiepin-3-carboxylate-5-oxide

540 mg. methyl-10,11-dihydrodibenzo[b,f]thiepin-3-carboxylate is dissolved in 40 cc methylene chloride and m-chloroperbenzoic acid is added in small portions until TLC shows very little starting material left. Excess of calcium hydroxide powder is added and the mixture filtered through celite. The organic layer is evaporated and the residue chromatographed on silica gel to afford 570 mg. (94.7%).

STEP 2

10,11-Dihydrodibenzo[b,f]thiepin-3-carboxylic Acid 5-oxide

520 mg. methyl-10,11-dihydrodibenzo[b,f]thiepin-3-carboxylate 5-oxide is stirred at 65°C. in a mixtureof 20 cc. 20% aqueous sodium hydroxide and 20 cc. ethanol until a clear solution results (1 hour). The mixture is diluted with water, extracted with ether, then acidified with hydrochloric acid. The white precipitate is filtered and dried to yield 245 mg. of material which is recrystallized from methanol to yield 180 mg., m.p. 261—265°C.

Example 9

10,11-Dihydrodibenzo[b,f]thiepin-3-carboxylic Acid 5,5-dioxide

735 mg. of 10,11-dihydrodibenzo[b,f]thiepin-3-carboxylic acid is heated at 75°—80°C. in 30 cc. acetic acid and 5 cc. 30% hydrogen peroxide for 2 hours. The reaction mixture is cooled down, the crystals filtered and washed with water and dried to yield 650 mg., m.p. 248°—250°C.

| | | | | | | |
|---|---|---|---|---|---|---|
| Calculated : | C: | 62.49; | H: | 4.20; | S: | 11.12. |
| Found : | C: | 62.11; | H: | 4.38; | S: | 10.97. |

Example 10

STEP 1

3,11-Dibromo-10,11-dihydrodibenzo[b,f]thiepin

10,5 g. 3-bromo-10,11-dihydro-11-hydroxydibenzo[b,f]thiepin and 25 cc. phosphorus tribromide are stirred for 2 hours, poured onto ice and extracted with ether. The ether is dried and evaporated to yield 10.65 g. of a brown oil which solidifies on standing. The material is used as such for the next step.

STEP 2

3-Bromo-10,11-dihydrodibenzo[b,f]thiepin

10.55 g. 3,11-dibromo-10,11-dihydrodibenzo[b,f]thiepin is suspended in 150 cc. sulfolane and gradually, in portions, 3.5 g. sodium borohydride is added. When the foaming has subsided, water is added and the mixture extracted four times with ether, the combined extracts are washed several times with water, dried and evaporated to dryness, and the residual oil chromatographed on silica gel. On elution with 10% benzene in hexane, 3.31 g. of pure material as a white solid is obtained, m.p. 69°—71°C.

STEP 3

3-Cyano-10,11-dihydrodibenzo[b,f]thiepin

4.61 g. 3-bromo-10,11-dihydrodibenzo[b,f]thiepin and 4.3 g. cuprous cyanide are refluxed in 50 cc. dimethylformamide for 5 1/2 hours. The mixture is poured onto ice and the solid filtered. The solid is triturated with chloroform and filtered. The chloroform is evaporated to yield 4.5 g. residue which is chromatographed on silica gel. Elution with benzene affords 2.83 g. (75.4%) of the desired pure oily compound which crystallizes on standing. Elution with ethyl acetate gives 440 mg. 10,11-dihydrodibenzo[b,f]thiepin-3-carboxylic acid amide as a white solid, m.p. 145°—147°C.

STEP 4

10,11-Dihydro-3-(5-tetrazolyl)dibenzo[b,f]thiepin

To 21 cc. dry tetrahydrofuran, cooled in an ice bath, 2.08 g. $AlCl_3$ is added, followed by 1.7 g. 3-cyano-10,11-dihydro[b,f]thiepin and 2.03 g. sodium azide. The resulting mixture is refluxed for 7 hours then cooled. 6.2 m 15% aqueous hydrochloric acid is added slowly. The reaction mixture is decanted, the residue extracted several times with ethyl acetate. The organics are combined, extracted several times with water, then once with sodium bicarbonate solution. The basic aqueous solution is acidified with hydrochloric acid. The tetrazole crystallizes out as white fluffy needles which are filtered and washed with water and dried, yield 1.065 g., m.p. 205°C.

| | | | | |
|---|---|---|---|---|
| Calculated : | C: 64.76; | H: 4.31; | N: 19.98; | S: 11.44. |
| Found : | C: 64.13; | H: 4.46; | N: 19.87; | S: 11.36. |

Example 11

10,11-Dihydro-3-(5-tetrazolyl)dibenzo[b,f]thiepin-5-oxide

A mixture of 600 mg. of 10,11-dihydro-3-(5-tetrazolyl)dibenzo[b,f]thiepin, 25 cc.acetic acid and 5 cc. 30% hydrogen peroxide is stirred for 6 hours. The suspended solid is filtered, washed with acetic acid, then water and dried to yield 575 mg. of pure material, m.p. 287°C. dec.

| | | | | |
|---|---|---|---|---|
| Calculated : | C: 60.79; | H: 4.08; | N: 18.91; | S: 10.82. |
| Found : | C: 60.65; | H: 4.28; | N: 18.77; | S: 10.59. |

Example 12

STEP 1

3-Cyano-10,11-dihydrodibenzo[b,f]thiepin-5,5-dioxide

800 mg. 3-cyano-10,11-dihydrodibenzo[b,f]thiepin is dissolved in 75 cc. methylene chloride and 1.73 g. m-chloroperbenzoic acid is added and the solution stirred at room temperature for three hours. An additional 10 cc. methylene chloride is added, followed by a large excess of powdered calcium hydroxide. After stirring for 5 minutes, the mixture is filtered through celite and the filtrate stripped to dryness and the residual solid triturated with ether and filtered to yield 854 mg. (94%) of the desired pure white solid.

STEP 2

10,11-Dihydro-3-(5-tetrazolyl)dibenzo[b,f]thiepin 5,5-dioxide

To 9 cc. tetrahydrofuran, cooled in an ice bath, are added in the following order 862 mg. aluminum chloride, 792 mg. 3-cyano-10,11-dihydrodibenzo[b,f]thiepin-5,5-dioxide, and 839 mg. sodium azide. The mixture is refluxed for six hours, cooled down and treated with 3 cc. 15% aqueous hydrochloric acid. The organic solvent is decanted and the residue triturated several times with ethyl acetate. The combined organic phases are washed with water, then extracted with aqueous sodium bicarbonate solution. The aqueous layer is acidified with hydrochloric acid and the precipitated tetrazole is filtered, washed with water, and dried, yield 580 mg. (63%), m.p. 233°C. dec.

| | | | | | | | | | | | | | | |
|---|---|---|---|---|---|---|---|---|---|---|---|---|---|---|
| Calculated | : | C: | 57.68; | H: | 3.87; | N: | 17.94; | S: | 10.27. |
| Found | : | C: | 57.53; | H: | 4.06; | N: | 17.66; | S: | 10.15. |

Example 13

STEP 1

2-(3′-Bromophenylthio)-5-nitrobenzoic Acid

A mixture of 205 g. 3-bromothiophenol, 188 g. potassium hydroxide in 2 l. water, 205 g. 2-chloro-5-nitrobenzoic acid and 6.5 g. copper powder is refluxed for two hours, filtered while hot, acidified with hydrochloric acid and the solid filtered, washed with water and dried to yield 301 g. of the desired white solid, m.p. 202°—206°C.

STEP 2

2-(3′-Bromophenylthio)-5-nitrobenzyl Alcohol

Prepared in 83% yield as described in Example I, Step 2, m.p. 98°—101°C.

STEP 3

2-(3′-Bromophenylthio)-5-nitrobenzyl Bromide

Prepared in 96% yield as described in Example I, Step 3, m.p. 80°—82°C.

STEP 4

2-(3′-Bromophenylthio)-5-nitrobenzyl Cyanide

2.16 g. 2-(3′-bromophenylthio)-5-nitrobenzyl bromide is refluxed in 50 cc. ethanol and 2.4 g. of cyanide ion exchange resin for 1/2 hour. The mixture is filtered hot and the resin washed with ethyl acetate. The combined organic filtrates are evaporated to dryness to yield 1.85 g. of crude residue which is chromatographed on silica gel. Elution with benzene affords 800 mg. of the desired compound.

STEP 5

2-(3′-Bromophenylthio)-5-nitroacetic Acid

2-(3′-Bromophenylthio)-5-nitrobenzyl cyanide is hydrolysed as described in Example I, Step 5, m.p. 123°—125°C.

STEP 6

3-Bromo-10,11-dihydro-8-nitro-11-oxodibenzo[b,f]thiepin

2-(3′-Bromophenylthio)-5-nitroacetic acid is transformed to the title compound in 95% yield as described in Example I, Step 6, m.p. 242°—245°C.

STEP 7

3-Bromo-10,11-dihydro-11-hydroxy-8-nitrodibenzo[b,f]thiepin

3-Bromo-10,11-dihydro-8-nitro-11-oxodibenzo[b,f]thiepin is reduced in 98% yield as described in Example I, Step 7.

STEP 8

3-Bromo-8-nitrodibenzo[b,f]thiepin

3-Bromo-10,11-dihydro-11-hydroxy-8-nitrodibenzo[b,f]thiepin is dehydrated as described in Example I, Steps 8, in 81% yield, m.p. 190°—192°C.

STEP 9

3-Cyano-8-nitrodibenzo[b,f]thiepin

3-Bromo-8-nitrodibenzo[b,f]thiepin is reacted with cuprous cyanide as described in Example I, Step 9, in 69.5% yield, m.p. 234°—236°C.

STEP 10

8-Nitrodibenzo[b,f]thiepin-3-carboxylic Acid

3-Cyano-8-nitrodibenzo[b,f]thiepin is hydrolyzed as described in Example I, Step 10, m.p. 304°—306°C.

| | | | | | | | | | |
|---|---|---|---|---|---|---|---|---|---|
| Calculated | : | C: | 60.19; | H: | 3.03; | N: | 4.68; | S: | 10.71. |
| Found | : | C: | 59.68; | H: | 3.08; | N: | 4.57; | S: | 10.75. |

Example 14

8-Nitrodibenzo[b,f]thiepin-3-carboxylic Acid 5-oxide

400 mg. 8-nitrodibenzo[b,f]thiepin-3-carboxylic acid is stirred for 24 hours in 200 cc. acetic acid and 1.5 cc. 30% hydrogen peroxide. The product is filtered, washed with water and dried to yield 315 mg. (74%) of pure product, m.p. 278°—281°C.

Example 15

STEP 1

3-Cyano-8-nitrodibenzo[b,f]thiepin-5,5-dioxide

2 g. 3-cyano-8-nitrodibenzo[b,f]thiepin is dissolved in 600 cc. chloroform and 12 g. m-chloroperbenzoic acid added. The solution is stirred for 24 hours. Excess powdered calcium hydroxide is added, the mixture filtered through celite, the filtrate evaporated to dryness and the residue chromatographed on silica gel. Elution with 5% ethyl acetate in benzene afforded 1.36 g. (61%) of pure sulfone.

STEP 2

8-Nitrodibenzo[b,f]thiepin-3-carboxylic Acid 5,5-dioxide

400 mg. 3-cyano-8-nitrodibenzo[b,f]thiepin-5,5-dioxide is refluxed in 5 cc. acetic acid and 5 cc. 50% aqueous sulfuric acid for 24 hours. The mixture is cooled down, the crystallized material is filtered, washed with water, dried at 135°C. in vacuum to yield 345 mg. (81.2%) acid, m.p. 310°—312°C.

| | | | | | | | | | |
|---|---|---|---|---|---|---|---|---|---|
| Calculated : | C: | 54.38; | H: | 2.72; | N: | 4.22; | S: | 9.68. |
| Found : | C: | 54.31; | H: | 2.97; | N: | 4.24; | S: | 9.69. |

Example 16

STEP 1

8-Amino-3-cyanodibenzo[b,f]thiepin

5.6 g. 3-cyano-8-nitrodibenzo[b,f]thiepin is dissolved in 600 cc. tetrahydrofuran and 27 g. stannous chloride dihydrate in 40 cc. water is added, followed by 100 cc. concentrated hydrochloric acid. The mixture is stirred for 24 hours, poured onto ice, and shaken with 300 cc. 20% sodium hydroxide. The organic layer is separated and the aqueous basic layer shaken once more with tetrahydrofuran. The combined organic extracts are dried and the solvent is evaporated to dryness and the residue chromatographed on silica gel and eluted with 20% ethyl acetate in benzene to yield 2.45 g. of the pure amine, m.p. 188°—190°C.

STEP 2

8-Aminodibenzo[b,f]thiepin-3-carboxylic Acid Hydrochloride Salt

850 mg. 8-amino-3-cyanodibenzo[b,f]thiepin is refluxed in a mixture of 20 cc. acetic acid and 20 cc. concentrated hydrochloric acid for 48 hours. The suspended solid is filtered, washed with some acetic acid, dried overnight under vacuum at 70°C. to yield 600 mg. pure compound, m.p. 308°—311°C.

| | | | | | | | | | | |
|---|---|---|---|---|---|---|---|---|---|---|
| Calculated : | C: | 58.92; | H: | 3.95; | N: | 4.58; | Cl: | 11.59; | S: | 10.48. |
| Found : | C: | 58.65; | H: | 4.17; | N: | 4.49; | Cl: | 11.66; | S: | 10.35. |

Example 17

STEP 1

8-Amino-3-cyanodibenzo[b,f]thiepin-5,5-dioxide

875 mg. 3-cyano-8-nitrodibenzo[b,f]thiepin-5,5-dioxide is dissolved in 105 cc. tetrahydrofuran. 15 ml. concentrated hydrochloric acid is added, followed by 3.8 g. stannous chloride dihydrate and the mixture is stirred for 24 hours. A solution of 20% sodium hydroxide is added, the mixture is shaken and the organic layer separated; the aqueous phase extracted once with ethyl acetate. The combined organic extracts are dried over sodium sulfate and evaporated to dryness. The residue is chromatographed on silica gel to yield 464 mg. (58%) pure amino compound, m.p. 321°—324°C.

STEP 2

8-Aminodibenzo[b,f]thiepin-3-carboxylic Acid 5,5-dioxide Hydrochloride

415 mg. 8-amino-3-cyanodibenzo[b,f]thiepin-5,5-dioxide is suspended in a mixture of 15 cc. concentrated hydrochloric acid and 15 cc. acetic acid and refluxed for 24 hours. The reaction mixture is evaporated to dryness and the residue collected and dried at 100°C. at 0.5 mm./Hg. to yield 454 mg. pure product, m.p. 280°C.

| | | | | | | | | | | |
|---|---|---|---|---|---|---|---|---|---|---|
| Calculated : | C: | 53.33; | H: | 3.58; | N: | 4.14; | Cl: | 10.49; | S: | 9.49. |
| Found : | C: | 53.30; | H: | 3.32; | N: | 4.28; | Cl: | 10.47; | S: | 9.65. |

Example 18

STEP 1

8-Chloro-3-cyanodibenzo[b,f]thiepin

1 g. 8-amino-3-cyanodibenzothiepin is suspended in 16 cc. concentrated hydrochloric acid and 5 cc. of water. The suspension is maintained at a temperature between 0 and 5°C. and 300 mg. sodium nitrite in 2 cc. of cold water is added slowly. The reaction mixture is stirred for 1/2 hour. A mixture of

400 mg. cuprous chloride and 700 mg. cupric chloride dihydrate is then added slowly. After the evolution of nitrogen has subsided, the reaction mixture is stirred at room temperature for 20 minutes, water added and the reaction mixture extracted with chloroform, dried over sodium sulfate and evaporated to dryness to yield 995 mg. (92%) of the pure chloro derivative, m.p. 207°—210°.

STEP 2

8-Chlorodibenzo[b,f]thiepin-3-carboxylic Acid

550 mg. 8-chloro-3-cyanodibenzo[b,f]thiepin is refluxed for 16 hours in a mixture of 15 cc. acetic acid and 15 cc. 50% aqueous sulfuric acid. The reaction mixture is cooled down and the product filtered, washed with water and dried to yield 485 mg. (82% of the pure acid, m.p. 302—304°C.

| | | | | |
|---|---|---|---|---|
| Calculated : | C: 62.39; | H: 3.14; | Cl: 12.27; | S: 11.10. |
| Found : | C: 62.48; | H: 3.06; | Cl: 12.01; | S: 10.89. |

Example 19

STEP 1

8-Chloro-3-cyanodibenzo[b,f]thiepin-5,5-dioxide

400 mg. 8-chloro-3-cyanodibenzo[b,f]thiepin is dissolved in 30 cc. chloroform and 2 g. m-chloroperbenzoic acid added, and the solution stirred for 24 hours. 5 g. calcium hydroxide is added, the mixture stirred for five minutes and filtered through celite. The organic filtrate is evaporated to dryness and the residue chromatographed on silica gel and eluted with 5% ethyl acetate/benzene mixture to yield 339 mg. (76%) pure sulfone, m.p. 228°—230°C.

STEP 2

8-Chlorodibenzo[b,f]thiepin-3-carboxylic Acid 5,5-dioxide

320 mg. 8-chloro-3-cyanodibenzo[b,f]thiepin-5,5-dioxide is refluxed for 24 hours in a mixture of 10 cc. acetic acid and 10 cc. 50% aqueous sulfuric acid. The mixture is cooled down and the crystalline product filtered, washed with water and dried at 70°C., 0.5 mm./Hg., to yield 289 mg. (82%) of pure acid, m.p. 276°—278°C.

| | | | | |
|---|---|---|---|---|
| Calculated : | C: 56.17; | H: 2.82; | Cl: 11.05; | S: 9.99. |
| Found : | C: 56.19; | H: 3.09; | Cl: 10.82; | S: 9.82. |

Example 20

8-Hydroxydibenzo[b,f]thiepin-3-carboxylic Acid

The diazonium chloride, prepared from 1 g. 8-amino-3-cyanodibenzo[b,f]thiepin, is heated at 90°C. for three to four hours in 50 cc. 50% aqueous sulfuric acid. The reaction mixture is cooled down, extracted with ethyl acetate, dried and evaporated and the residue chromatographed on silica gel and eluted with a mixture of ammonium hydroxide, chloroform, methanol, in the ratio 1:8:4 by volume to yield 250 mg. of the pure phenol.

Example 21

8-Hydroxydibenzo[b,f]thiepin-3-carboxylic Acid 5,5-dioxide

The diazonium chloride, prepared from 1 g. 8-amino-3-cyanodibenzo[b,f]thiepin-5,5-dioxide, is treated in Example 22 to yield 300 mg. of the desired compound.

Example 22

8-Mercaptodibenzo[b,f]thiepin-3-carboxylic Acid

A cold solution of the diazonium chloride (see Example 20) is added fairly quickly to a solution of 1 g. potassium thioxanthate in 15 cc. water, maintained at 45°—65°C. At the end of the addition, 10 cc. 40% potassium hydroxide and 10 cc. ethanol are added and the reaction mixture is refluxed for three hours, cooled down, acidified with hydrochloric acid and extracted with ethyl acetate. The organic extract is dried, evaporated to dryness and the residue chromatographed on silica gel and eluted with a mixture of ammonium hydroxide, chloroform, methanol, in the ratio of 1:8:4, to yield 500 mg. of the mercapto acid.

Example 23

8-Mercaptodibenzo[b,f]thiepin-3-carboxylic Acid 5,5-dioxide

A cold solution of the diazonium chloride of Example 20 is treated in an identical fashion to Example 22 to yield 450 mg. of the desired compound.

Example 24

8-Methoxydibenzo[b,f]thiepin-3-carboxylic Acid

1 g. 8-hydroxydibenzo[b,f]thiepin-3-carboxylic acid is stirred in 50 cc. dimethylformamide with 1.5 g. potassium carbonate and 3 cc. methyl iodide. 100 ml. water is added to the reaction mixture,

which is heated for three hours and then extracted with ethyl acetate, and the aqueous layer is acidified. The precipitated solid is filtered, washed with water and a small volume of cold methanol, and dried. Yield of the desired material 700 mg.

Example 25

8-Methylthiodibenzo[b,f]thiepin-3-carboxylic Acid

1 g. 8-mercaptodibenzo[b,f]thiepin-3-carboxylic acid is treated as in Example 24 to yield 650 mg. of the desired compound.

Example 26

8-Methoxydibenzo[b,f]thiepin-3-carboxylic Acid 5,5-dioxide

1 g. 8-hydroxydibenzo[b,f]thiepin-3-carboxylic 5,5-dioxide acid is treated as in Example 24 to yield 830 mg. product.

Example 27

8-Methylthiodibenzo[b,f]thiepin-3-carboxylic Acid 5,5-dioxide

1 g. 8-mercaptodibenzo[b,f]thiepin-3-carboxylic acid 5,5-dioxide is treated as in Example 24 to yield 650 mg. of the desired compound.

Example 28

8-Methylsulfinyldibenzo[b,f]thiepin-3-carboxylic Acid 5,5-dioxide

500 mg. 8-methylthiodibenzo[b,f]thiepin-3-carboxylic acid in 15 cc. acetic acid and 3 cc. 30% hydrogen peroxide is stirred at room temperature for 8 hours. 15 ml. water is added to the reaction mixture and the suspended solid filtered and dried to afford 450 mg. of the desired compound.

Example 29

8-Methylsulfinyldibenzo[b,f]thiepin-3-carboxylic Acid 5,5-dioxide

450 mg. 8-methylthiodibenzo[b,f]thiepin-3-carboxylic acid 5,5-dioxide is stirred at room temperature in 15 cc. acetic acid and 1.5 cc. 30% hydrogen peroxide for an 8-hour period. Water is added and the white solid filtered, washed with water and dried to afford 400 mg. of the desired compound.

Example 30

8-Methylsulfonyldibenzo[b,f]thiepin-3-carboxylic Acid 5,5-dioxide

1 g. 8-methylthiodibenzo[b,f]thiepin-3-carboxylic acid 5,5-dioxide is heated at 80°—100°C. in 15 cc. acetic acid and 3 cc. 30% hydrogen peroxide. The reaction mixture is cooled down, water added, and the solid filtered and dried to yield 800 mg. of product.

Example 31

8-Acetyl-3-cyano-10,11-dihydrodibenzo[b,f]thiepin

Add 125.5 g. (0.94 mole) of alumin chloride and 47.9 g. (0.47 mole) of acetic anhydride to 1.01 of dichloroethane. Stir at room temperature for 10 minutes. Add 14 g. (0.058 mole) of 3-cyano-10,11-dihydrodibenzo[b,f]thiepin in portions. Stir at room temperature for 4 hours. Pour the reaction mixture over ice and extract into ethyl acetate. Wash with water, dry over sodium sulfate, filter and evaporate to dryness to obtain the title product. (m.p. 138°—141°.)

Example 32

8-Acetyl-10,11-dihydrodibenzo[b,f]thiepin-3-carboxylic Acid

Add 8.5 g. of the nitrile of Example 33 to a mixture of 220 ml. of acetic acid (glacial) and 220 ml. of 50% sulfuric acid. Heat the mixture at reflux under a nitrogen atmosphere overnight. Cool to room temperature and separate the solids by filtration. Recrystallize from acetic acid to obtain the title product. (m.p. 230°—232°C.)

Example 33

8-Acetyl-3-cyano-10,11-dihydrodibenzo[b,f]thiepin-5,5-dioxide

Dissolve 2.5 g. of 8-acetyl-3-cyano-10,11-dihydrodibenzo[b,f]thiepin in 200 cc. of methylene chloride and add 6.0 g. of m-chloroperbenzoic acid. Stir at room temperature overnight. Add 12.0 g. of calcium hydroxide and filter through celite. Evaporate to dryness to obtain the title product. (Yield 2.58 g. — 92.5%)

Example 34

8-Acetyl-10,11-dihydrodibenzo[b,f]thiepin-3-carboxylic Acid 5,5-Dioxide

Suspend 2.2 g. of 8-acetyl-3-cyano-10,11-dihydrodibenzo[b,f]thiepin-5,5-dioxide in 60 ml. of acetic acid and 60 ml. of 50% sulfuric acid. Reflux under a nitrogen atmosphere for 6 hours. Cool to room temperature. Evaporate the acetic acid, dilute with water and separate the solids by filtration.

Wash with water and air dry. Suspend the solid in ether (200 ml.), stir at room temperature and filter to obtain the title product. (m.p. 219°—221°C.)

Example 35

8-Acetamido-10,11-dihydrodibenzo[b,f]thiepin-3-carboxylic Acid

STEP 1

Oxime of 8-Acetyl-10,11-dihydrodibenzo[b,f]thiepin-3-carboxylic Acid

Reflux for 24 hours a mixture of 130 mg. of 8-acetyl-10,11-dihydrobenzo[b,f]thiepin-3-carboxylic acid, 180 mg. of hydroxylamine hydrochloride and 426 mg. of sodium acetate in 10 cc. of ethanol. Dilute with water and separate the solids by filtration. Wash with water and dry in order to obtain the title product.

STEP 2

8-Acetamido-10,11-dihydrodibenzo[b,f]thiepin-3-carboxylic Acid

Reflux 100 mg. of the oxime from Step 1 in 2 cc. of trifluoroacetic acid for 2 hours. Evaporate to dryness. Add water and extract into ether. Dry and evaporate to dryness to obtain the title product.

Example 36

8-Amino-10,11-dihydrodibenzo[b,f]thiepin-3-carboxylic Acid

Reflux 200 mg. of 8-acetamido-10,11-dihydrodibenzo[b,f]thiepin-3-carboxylic acid for 16 hours in a mixture of 5 cc. of acetic acid and 5 cc. of 50% aqueous sulfuric acid. Dilute with water and separate the solids by filtration. Wash with water and dry to obtain the title product.

Example 37

10,11-Dihydrodibenzo[b,f]thiepin-3,8-dicarboxylic Acid 5,5-Dioxide

Add 1.85 g. of 8-acetyl-10,11-dihydrodibenzo[b,f]thiepin-3-carboxylic acid to a stirred mixture of 370 ml. of 50% aqueous sodium hypochlorite and 13 ml. of 20% aqueous sodium hydroxide at 60°C. Raise the temperature to 85°C. and stir for 30 minutes. Pour the mixture over ice and add sodium metabisulfite. Acidify with 6N hydrochloric acid. Separate the solids by filtration wash with water and dry to obtain the title product. (m.p. 350°—352°C.)

Example 38

Dibenzo[b,f]thiepin-3,8-dicarboxylic Acid 5,5-Dioxide

Add 8.5 g. of the nitrile of Example 33 to a mixture of 220 ml. of acetic acid (glacial) and 220 ml. 50% sodium hypochlorite (400 ml.) and 15 ml. of 20% aqueous sodium hydroxide at 60°C. Heat for 48 hours at 75°C. Cool to room temperature and add sodium metabisulfite. Acidify and separate the solids by filtration. Purify by chromotography over silica gel eluting with a mixture of 80 parts toluene, 20 parts dioxane and 4 parts acetic acid. (m.p. ~375°C.)

The compounds of formula I are useful in the treatment or prophylaxis of mammalian disease conditions where excessive undesirable contractile activity of prostaglandins, such as $PGF_{2\alpha}$, or prostaglandin biosynthetic intermediates contribute. These conditions include asthma, inflammatory states such as arthritis, allergy, diarrhea, hypertension, angina, platelet aggregation, cerebral spasm, premature abortion and dismenorrhea. In particular, they are of value in reaginic mediated asthma (extrinsic asthma).

The magnitude of a prophylactic or therapeutic dose of compound of formula I will, of course, vary with the nature and the severity of the condition to be treated and with the particular compound of formula I and its route of administration. In general, the dose range lies within the range of 0.2 mg. to 100 mg. per kg. body weight of a mammal.

The pharmaceutical compositions of the present invention comprise a compound of formula I as an active ingredient, and may also contain pharmaceutically acceptable carrier and optionally other therapeutic ingredients. The compositions include compositions suitable for oral, rectal, opthalmic, pulmonary, nasal, dermal, topical or parenteral (including subcutaneous, intramuscular and intravenous) administration, although the most suitable route in any given case will depend on the nature and severity of the condition being treated and on the nature of the active ingredient. They may be conveniently presented in unit dosage form and prepared by any of the methods well known in the art of pharmacy.

For use where a composition for intravenous administration is employed, a suitable dosage range is from 0.2 to 10 mg. (preferably 1 to 5 mg.) of a compound of formula I per kg. of body weight per day and in the case where an oral composition is employed a suitable dosage range of about, e.g., 1 to 50 mg. of a compound of formula I per kg. of body weight per day, preferably from 10 to 40 mg./kg.

Pharmaceutical compositions of the present invention suitable for oral administration and by inhalation in the case of asthma therapy may be presented as discrete units such as capsules, cachets or tablets each containing a predetermined amount of the active ingredient; as a powder or granules; or as a solution or a suspension in an aqueous liquid, a non-aqueous liquid, an oil-in-water emulsion or a water-in-oil liquid emulsion. Such compositions may be prepared by any of the methods of pharmacy

but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or moulding, optionally with one or more accessory ingredients. Compressed tablets may be prepared by compressing in a suitable machine, the active ingredient in a free-flowing form such as powder or granules, optionally mixed with a binder, lubricant, inert diluent or surface active or dispersing agent. Moulded tablets may be made by moulding in a suitable machine, a mixture of the powdered compound moistened with an inert liquid diluent. Desirably, each tablet contains from 50 mg. to 500 mg. of the active ingredient and each cachet or capsule contains from 50 mg. to 500 mg. of the active ingredient.

Although the invention has been described in the foregoing specification in terms of the use of the novel thiepin disclosed herein in the treatment and control of human and warm-blooded animal disease conditions characterized by excessive undesirable contractile activity of prostaglandins and prostaglandin biosynthetic intermediates, and particularly of asthma, it will be recognized by those skilled in the art that, in addition to the involvement of contractile prostaglandins in chronic obstructive lung disease (e.g., asthma), prostaglandins play a role in other allergic conditions as well as in inflammation, diarrhea, hypertension, angina, cerebral spasm, premature abortion and dismenorrhea. Also, the thiepins of this invention are potent $TXA_2$ biosynthesis inhibitors, inhibiting platelate aggregation, and can be useful in diseases such as atherosclerosis, variant anginal and myocardial infarction.

**Claims**

1. A compound of the formula:

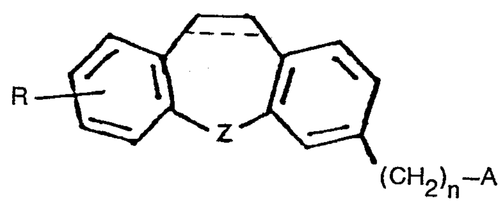

in which *n* is 0 or an integer from 1 to 4; Z is thio, sulfinyl, or sulfonyl; R in the 7- or 8-position and is hydrogen, halogen, amino, $C_{1-4}$ alkyl, $C_{1-4}$ alkanoyl, hydroxyl, $C_{1-4}$ alkoxy, mercapto, $C_{1-4}$ alkylthio, $C_{1-4}$ alkylsulfinyl, $C_{1-4}$ alkylsulfonyl, trifluoromethyl, trifluoromethylthio, cyano, carboxy, nitro, $C_{1-4}$ alkylamino or di($C_{1-4}$ alkyl)amino; A is 5-tetrazolyl, 3-hydroxy-1,2,5-thiadiazol-4-yl, 4-hydroxy-2,5-dioxo-$\Delta^3$-pyrrolin-3-yl or

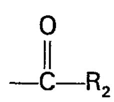

where $R_2$ is hydroxy, $C_{1-4}$ alkoxy, N,N-di($C_{1-4}$ alkyl)amino-($C_{1-4}$ alkoxy), $C_{1-4}$ hydroxyalkoxy, carboxy-($C_{1-4}$ alkoxy), amino, $C_{1-4}$ alkylamino, di($C_{1-4}$ alkyl)amino, $C_{1-4}$ alkylsulfonylamino, carboxy($C_{1-4}$ alkyl)amino, carbamoyl($C_{1-4}$ alkyl)amino or 2-imino-3-methylthiazolidine and the dotted line indicates either an olefinic bond or saturation at the 10,11-position; and the pharmaceutically acceptable salts thereof.

2. A compound according to Claim 1 in which *n* is 1 to 4 and A is carboxy.
3. A compound according to Claim 1 or 2 in which R is chloro.
4. A compound according to Claim 1 or 2 in which R is acetyl.
5. Dibenzo[b,f]thiepin-3-carboxylic acid.
6. Dibenzo[b,f]thiepin-3-carboxylic acid-5-oxide.
7. Dibenzo[b,f]thiepin-3-carboxylic acid 5,5-dioxide.
8. 3-(5-Tetrazolyl)dibenzo[b,f]thiepin 5,5-dioxide.
9. 3-(5-Tetrazolyl)dibenzo[b,f]thiepin.
10. 3-(5-Tetrazolyl)dibenzo[b,f]thiepin-5-oxide.
11. 10,11-Dihydrodibenzo[b,f]thiepin-3-carboxylic acid.
12. 10,11-Dihydrodibenzo[b,f]thiepin-3-carboxylic acid 5-oxide.
13. 10,11-Dihydrodibenzo[b,f]thiepin-3-carboxylic acid 5,5-dioxide.
14. 10,11-Dihydro-3-(5-tetrazolyl)dibenzo[b,f]thiepin.
15. 10,11-Dihydro-3-(5-tetrazolyl)dibenzo[b,f]thiepin-5-oxide.
16. 10,11-Dihydro-3-(5-tetrazolyl)dibenzo[b,f]thiepin-5,5-dioxide.
17. A process for preparing a compound of the formula:

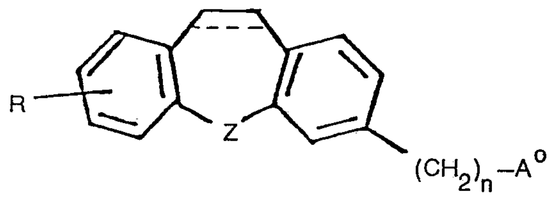

in which Z, R, the dotted line and *n* are as defined in Claim 1 and A° is 5-tetrazolyl or carboxy, that comprises hydrolysing a compound of the formula:

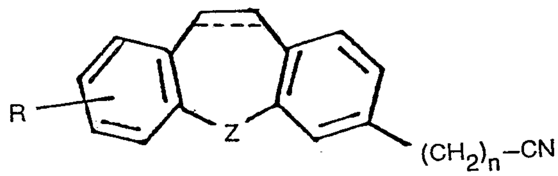

with an acid or base to form the carboxyl compound or reacting the said compound with an azide ion to form the 5-tetrazolyl compound.

18. A composition for treating undesirable contractile activity of prostaglandins consisting essentially of a pharmaceutically acceptable carrier and a therapeutically effective amount of a compound as claimed in any one of Claims 1 to 16.

**Revendications**

1. Composé de la formule:

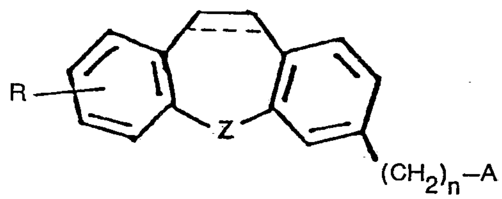

dans laquelle *n* est 0 ou un nombre entier de 1 à 4; Z est un groupe thio, sulfinyle ou sulfonyle; R se trouve dans la position 7 ou 8 et est de l'hydrogène, un halogène, un groupe amino, alcoyle en $C_{1-4}$, alcanoyle en $C_{1-4}$, hydroxyle, alcoxy en $C_{1-4}$, mercapto (alcoyl en $C_{1-4}$) thio, (alcoyl en $C_{1-4}$) sulfinyle, (alcoyl en $C_{1-4}$) sulfonyle, trifluorométhyle, trifluorométhylthio, cyano, carboxy, nitro, (alcoyl en $C_{1-4}$) amino ou di(alcoyl en $C_{1-4}$) amino; A est un groupe 5-tétrazolyle, 3-hydroxy-1,2,5-thiadiazol-4-yle, 4-hydroxy-2,5-dioxo-$\Delta^3$-pyrrolin-3-yle ou

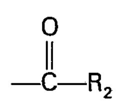

où R est un groupe hydroxy, alcoxy en $C_{1-4}$, N,N-di(alcoyl en $C_{1-4}$)amino-(alcoxy en $C_{1-4}$), hydroxyalcoxy en $C_{1-4}$, carboxy-(alcoxy en $C_{1-4}$), amino, (alcoyl en $C_{1-4}$) amino, di(alcoyl en $C_{1-4}$) amino, (alcoyl en $C_{1-4}$) sulfonylamino, carboxy(alcoyl en $C_{1-4}$) amino, carbamoyl (alcoyl en $C_{1-4}$) amino ou 2-imino-3-méthylthiazolidine et la ligne en pointillé indique soit une liaison oléfinique soit une saturation à la position 10,11; et ses sels pharmaceutiquement acceptables.

2. Composé selon la revendication 1, caractérisé en ce que *n* est un nombre de 1 à 4 et A est un groupe carboxy.

3. Composé selon l'une des revendications 1 et 2, caractérisé en ce que R est du chlore.

4. Composé selon l'une des revendications 1 et 2, caractérisé en ce que R est un groupe acétyle.

5. Acide dibenzo(b,f)thiépine-3-carboxylique.

6. 5-oxyde d'acide dibenzo(b,f)thiépine-3-carboxylique.

7. 5,5-dioxyde d'acide dibenzo(b,f)thiépine-3-carboxylique.

8. 5,5-dioxyde de 3-(5-tétrazolyl)dibenzo(b,f)thiépine.

9. 3-(5-tétrazolyl)dibenzo(b,f)thiépine.

10. 5-oxyde de 3-(5-tétrazolyl)dibenzo(b,f)thiépine.

11. Acide 10,11-dihydrodibenzo(b,f)thiépine-3-carboxylique.
12. 5-oxyde d'acide 10,11-dihydrodibenzo(b,f)thiépine-3-carboxylique.
13. 5,5-dioxyde d'acide 10,11-dihydrodibenzo(b,f)thiépine-3-carboxylique.
14. 10,11-dihydro-3-(5-tétrazolyl)dibenzo(b,f)thiépine.
15. 5-oxyde de 10,11-dihydro-3-(5-tétrazolyl)dibenzo(b,f)thiépine.
16. 5,5-dioxyde de 10,11-dihydro-3-(5-tétrazolyl)dibenzo(b,f)thiépine.
17. Procédé pour la préparation d'un composé de la formule:

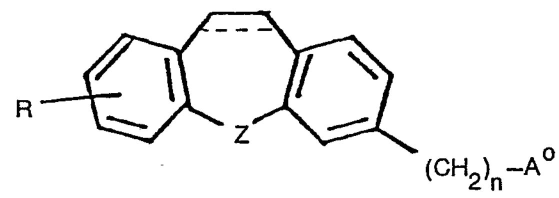

dans laquelle Z, R, la ligne en pointillé et *n* sont tels que définis dans la revendication 1 et A° est un groupe 5-tétrazolyle ou carboxy, caractérisé en ce qu'on hydrolyse un composé de la formule:

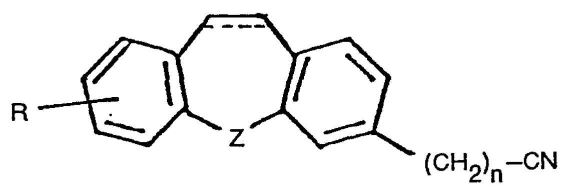

avec un acide ou base de manière à former le composé carboxylé ou on fait réagir ce composé avec un ion azide de manière à former le composé 5-tétrazolyle.

18. Composition pour le traitement de l'activité contractile indésirable de prostaglandines, constituée essentiellement d'un véhicule pharmaceutiquement acceptable et d'un quantité thérapeutiquement efficace d'un composé selon l'une quelconque des revendications 1 à 16.

**Patentansprüche**

1. Verbindung der Formel:

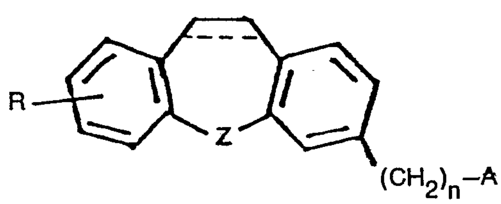

in welcher bedeuten: *n* 0 oder eine Zahl von 1 bis 4; Z Thio, Sulfinyl oder Sulfonyl; R, welches sich in der 7- oder 8-Stellung befinden kann, Wasserstoff, Halogen, Amino, $C_{1-4}$-Alkyl, $C_{1-4}$-Alkanoyl, Hydroxyl, $C_{1-4}$-Alkoxy, Mercapto, $C_{1-4}$-Alkylthio, $C_{1-4}$-Alkylsulfinyl, $C_{1-4}$-Alkylsulfonyl, Trifluoromethyl, Trifluoromethylthio, Cyano, Carboxy, Nitro, $C_{1-4}$-Alkylamino oder Di-($C_{1-4}$-alkyl)amino; A 5-Tetrazolyl, 3-Hydroxy-1,2,5-thiadiazol-4-yl, 4-Hydroxy-2,5-dioxy-$\Delta^3$-pyrrolin-3-yl, oder

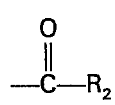

wobei $R_2$ bedeutet: Hydroxy, $C_{1-4}$-Alkoxy, N,N-Di-($C_{1-4}$-alkyl)-amino-($C_{1-4}$-alkoxy), $C_{1-4}$-Hydroxyalkoxy, Carboxy($C_{1-4}$-alkoxy), Amino, $C_{1-4}$-Alkylamino, Di-($C_{1-4}$-alkyl)-amino, $C_{1-4}$-Alkylsulfonylamino, Carboxy-($C_{1-4}$-alkyl)-amino, Carbamoyl-($C_{1-4}$-alkyl)-amino oder 2-Imino-3-methylthiazolidin, wobei die punktförmige Linie entweder eine olefinische Bindung oder Gesättigtheit in der 10,11-Stellung bedeutet; und deren pharmazeutisch annehmbare Salze.

2. Verbindung nach Anspruch 1, in welcher *n* 1 bis 4 und A Carboxy bedeuten.
3. Verbindung nach Anspruch 1 oder 2, in welcher R Chloro bedeutet.
4. Verbindung nach Anspruch 1 oder 2, in welcher R Acetyl bedeutet.
5. Dibenzo[b,f]thiepin-3-carbonsäure.
6. Dibenzo[b,f]thiepin-3-carbonsäure-5-oxid.

7. Dibenzo[b,f]thiepin-3-carbonsäure-5,5-dioxid.
8. 3-(5-Tetrazolyl)-dibenzo[b,f]thiepin-5,5-dioxid.
9. 3-(5-Tetrazolyl)-dibenzo[b,f]thiepin.
10. 3-(5-Tetrazolyl)-dibenzo[b,f]thiepin-5-oxid.
11. 10,11-Dihydrodibenzo[b,f]thiepin-3-carbonsäure.
12. 10,11-Dihydrodibenzo[b,f]thiepin-3-carbonsäure-5-oxid.
13. 10,11-Dihydrodibenzo[b,f]thiepin-3-carbonsäure-5,5-dioxid.
14. 10,11-Dihydro-3-(5-tetrazolyl)-dibenzo[b,f]thiepin.
15. 10,11-Dihydro-3-(5-tetrazolyl)-dibenzo[b,f]thiepin-5-oxid.
16. 10,11-Dihydro-3-(5-tetrazolyl)-dibenzo[b,f]thiepin-5,5-dioxid.
17. Verfahren zur Herstellung einer Verbindung der Formel:

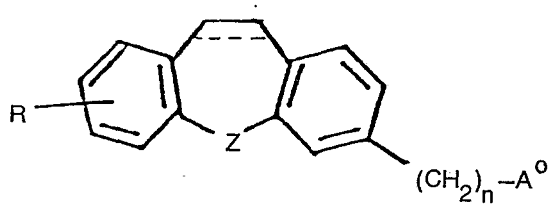

in welcher Z, R, die punktförmige Linie und *n* wie in Anspruch 1 definiert sind und A° 5-Tetrazolyl oder Carboxy bedeutet, welches die Schritte umfasst: Hydrolysieren einer Verbindung der Formel:

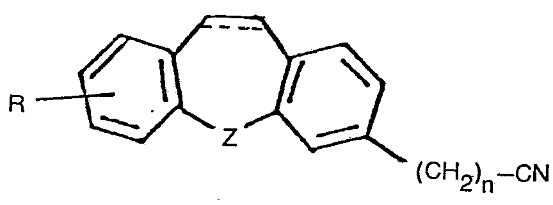

mit einer Säure oder Base, um die Carboxylverbindung zu bilden, oder Umsetzen der genannten Verbindung mit einem Azidion, um die 5-Tetrazolylverbindung zu bilden.

18. Zusammensetzung zur Behandlung unerwünschter Kontraktionswirksamkeit von Prostaglandinen, bestehend im wesentlichen aus einem pharmazeutisch annehmbaren Träger und einer therapeutisch wirksamen Menge einer Verbindung nach irgendeinem der Ansprüche 1 bis 16.